# EUROPEAN PATENT APPLICATION

(11) **EP 4 310 088 A1**
(43) Date of publication of application: **24.01.2024**
(21) Application number: 21905795.7
(22) Date of filing: 16.12.2021
(51) Int. Cl.: C07D 487/04, A61P 19/02, A61P 35/00, A61P 37/00, A61K 31/4188, A61K 31/4985

(54) **HETEROCYCLIC JAK INHIBITOR**

(30) Priority: 18.12.2020 CN 202011495497
(71) Applicant: Beijing InnoCare Pharma Tech Co., Ltd., Beijing 102206 (CN)
(72) Inventor: CHEN, Xiangyang, Beijing 102206 (CN); PANG, Yucheng, Beijing 102206 (CN)
(74) Representative: Patentanwälte Magenbauer & Kollegen Partnerschaft mbB
(86) International application number: PCT/CN2021/138842
(87) International publication number: WO 2022/127869

(57) **Abstract**

The present invention relates to a heterocyclic compound as a Janus kinase (JAK) inhibitor or a pharmaceutically acceptable salt thereof. Specifically, the present invention relates to a compound as represented by general formula (I) or a pharmaceutically acceptable salt thereof. The present invention also relates to a process for preparing the compound or a pharmaceutically acceptable salt thereof. The compounds of the present invention can be used for treating and/or preventing JAK-mediated related diseases, in particular inflammatory diseases, autoimmune diseases, and cancers. The definition of each substituent in the general formula (I) is the same as that in the description.

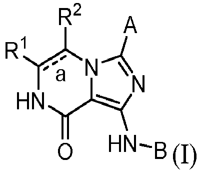

## Description

### Technical Field

The present invention relates to a heterocyclic compound for regulating or inhibiting Janus kinase (JAK) activity or a pharmaceutically acceptable salt thereof. The present invention also relates to a process for preparing the compound or a pharmaceutically acceptable salt thereof. The present invention further relates to the use and application of the compound or a pharmaceutically acceptable salt thereof in treating and/or preventing inflammatory diseases, autoimmune diseases, and cancers.

### Background technology

Janus kinase (JAK) is a non-receptor tyrosine-protein kinase composed of four family members, namely: JAK1, JAK2, JAK3, and TYK2. JAK has 7 homology domains in structure (JAK homology domain, JH), of which the JH1 domain is a kinase domain, the JH2 domain is a pseudo-kinase domain (which regulates the kinase activity of JH1), and JH6 and JH7 are receptor binding domains. When the cell surface area of the cytokine receptor is bound to cytokine, its intracellular area where JAKs are bound is phosphorylated, thereby creating a docking site for the signal transducer and activator of transcription proteins (STATs). The STAT proteins are further phosphorylated by activated JAKs to form a dimer, which enters the nucleus, regulates the expression and transcription of related genes, and enables signal transduction from the cell membrane to the nucleus (Lionard et. al., Ann. Rev. Immunol. 1998, 16, 293-322). Therefore, JAK transduces cytokine-mediated signals through the JAK-STAT pathway and plays an important role in many cellular functions such as cytokine-dependent regulation of cell proliferation, differentiation, apoptosis, and immune response, and is a popular target for the treatment of inflammatory diseases, autoimmune diseases, and cancers (Alicea-Velazquez et. al., Curr. Drug Targets 2011, 12, 546-55). Several pharmaceuticals as JAK inhibitors have been approved for marketing, including JAK1/JAK2 inhibitor ruxolitinib and JAK2 inhibitor fedratinib for treating myelofibrosis, and pan-JAK inhibitor tofacitinib, JAK1/JAK2 inhibitor baricitinib, pan-JAK inhibitor peficitinib and JAK1 inhibitor upadacitinib for treating rheumatoid arthritis, etc.

JAKs and STATs play a highly specific role in controlling different immune responses. A JAK enzyme can participate in the signal transduction processes induced by multiple cytokines, and a cytokine signaling pathway can also activate multiple JAK enzymes, but the cytokine itself has certain selectivity for STAT activation. For example, interleukin-4 (IL-4) activates STAT1/3/5/6, while IL-12 specifically activates STAT4. JAK1, JAK2, and TYK2 are widely present in various tissues and cells. JAK1 is closely related to the activation of inflammatory factors such as IL-6 and interferon (IFN), so the JAK1 selective inhibitor is considered to have a potential therapeutic effect on autoimmune diseases such as rheumatoid arthritis (RA) and psoriasis. JAK2 can independently mediate the signal transduction of cytokines such as erythropoietin (EPO) and thrombopoietin (TPO) (Won et. al., BMC Bioinformatics 2009, 10, S53), and is closely related to the proliferation and differentiation of blood cells. TYK2 is involved in the signal transduction of inflammatory cytokines such as interferons (IFNs), IL-12, and IL-23, and plays a key role in congenital immunity and adaptive immunity. Therefore, TYK2 has received great attention as a drug target for autoimmune diseases. For example, TYK2 inhibitors can be used for potential treatment of psoriasis, systemic lupus erythematosus (SLE), inflammatory bowel disease (IBD), etc. JAK3 is present only in the bone marrow and lymphatic system and mediates the signal transduction of IL-2, IL-4, IL-7, IL-9, IL-15, and IL-21. These cytokines play an important role in inducing the proliferation and differentiation of T cells, activating B cells to produce antibodies, activating macrophages, enhancing the activity of natural killer cells (NK cells), and inducing other cytokines such as IFN. Therefore, JAK3 selective inhibitor is expected to play an important role in organ transplantation and treating autoimmune diseases and inflammatory pneumonia.

Inflammatory bowel disease (IBD) is a common chronic intestinal tract inflammatory disease, including Crohn's disease (CD), ulcerative colitis (UC), and IBD unclassified (ibdu). Inflammatory bowel disease affects 500 million people worldwide and the prevalence increases year by year. The clinical symptoms are diarrhea, hematochezia, abdominal pain, fatigue, high fever, and the like, and common therapeutic drugs comprise 5-aminosalicylic acid (5-ASAs), glucocorticoid, immunosuppressant (such as azathioprine), and biological agents (such as anti-TNF, IL-12/IL-23 monoclonal antibody) and the like, but many treated patients are not relieved, and up to 80% of patients suffering from Crohn's disease and 30% of patients suffering from UC finally need to undergo surgery. There is also a great unmet medical need in this field, requiring more effective and safe drugs.

During the onset of the disease, the expression of proinflammatory factors such as IL-13 and IL-17 is increased, thereby inducing inflammation through the JAK-STAT pathway. The JAK inhibitor has potential application in treating CD and UC as a novel oral small molecule drug, wherein tofacitinib is approved to be used for treating UC in multiple countries, but the non-selective inhibition of JAK1/2/3 by tofacitinib causes relatively serious side effects, such as serious infection and malignant tumor initiation. However, due to the high sequence similarity of the catalytically active sites of JAK enzyme family members, it is quite difficult to design an oral selective JAK inhibitor with the systemic exposure required for treatment. Therefore, a novel JAK inhibitor is developed to enrich the local exposure required for treatment only at the site of action (such as the colon, skin, etc.), but with little blood solubility to avoid systemic adverse reactions, thereby exerting drug efficacy and improving safety. TD-1473 developed by Theravance is a pan-JAK inhibitor with intestinal local absorption. It has entered a clinical phase III trial and has shown good tolerance.

### Summary of the Invention

### Definition

Unless otherwise stated to the contrary, the following terms used in the specification and claims have the following meanings.

"C_{x-y}" represents the range of carbon atoms, wherein x and y are integers, for example, C₃₋₈cycloalkyl represents a cycloalkyl with 3-8 carbon atoms, that is, a cycloalkyl with 3, 4, 5, 6, 7 or 8 carbon atoms. It should also be understood that "C₃₋₈" also includes any subranges therein, for example, C₃₋₇, C₃₋₆, C₄₋₇, C₄₋₆, C₅₋₆, and the like.

"Alkyl" refers to a saturated straight or branched chain hydrocarbyl group containing 1 to 20 carbon atoms, for example, 1 to 8 carbon atoms, 1 to 6 carbon atoms, or 1 to 4 carbon atoms. Non-limiting examples of alkyl include methyl, ethyl, n-propyl, iso-propyl, n-butyl, iso-butyl, *tert*-butyl, *sec*-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, and the like.

"Cycloalkyl" refers to a saturated cyclic hydrocarbyl substituent containing 3 to 14 carbon ring atoms. Cycloalkyl can be a monocyclic carbon ring, typically containing 3 to 8, 3 to 7, or 3 to 6 carbon ring atoms. Non-limiting examples of monocyclic cycloalkyl include cyclopropyl, cyclobutyl, cyclopentyl, cyclohexyl, cycloheptyl, and the like. Cycloalkyl can also be a bi-or tricyclic ring that is fused, bridged, or spiro, such as decahydronaphthalenyl, bicyclo[2.2.2]octane, spiro[3.3]heptane, and the like.

"Heterocyclyl or heterocycle" refers to a saturated or partially unsaturated monocyclic or polycyclic cyclic group comprising 3 to 20 ring atoms, for example, 3 to 14, 3 to 12, 3 to 10, 3 to 8, 3 to 6, or 5 to 6 ring atoms, one or more of which are selected from nitrogen, oxygen, or S(O)ₘ (where m is an integer of 0 to 2), but excluding ring moieties of-O-O-, -O-S-, or-S-S-, the remaining ring atoms being carbon. Preferably it comprises 3 to 12 ring atoms, more preferably 3 to 10 ring atoms, more preferably 4 to 7 ring atoms, more preferably 4 to 6 ring atoms, most preferably 5 or 6 ring atoms, of which 1 to 4 are heteroatoms, more preferably of which 1 to 3 are heteroatoms, and most preferably of which 1 to 2 are heteroatoms. Non-limiting examples of monocyclic heterocyclyl include pyrrolidinyl, oxetanyl, piperidinyl, piperazinyl, tetrahydrofuranyl, tetrahydropyranyl, tetrahydrothiopyranyl, morpholinyl, thiomorpholinyl, homopiperazinyl, azetidinyl, and the like. Non-limiting examples of polycyclic heterocyclyl include fused, bridged or spiro polycyclic heterocyclic groups, such as octahydrocyclopenta[c]pyrrole, octahydropyrrolo[1,2-a]pyrazine, 3,8-diazabicyclo[3.2.1]octane, 5-azaspiro[2.4]heptane, 2-oxa-7-azaspiro[3.5]nonane, and the like.

"Aryl or aromatic ring" refers to an aromatic monocyclic or a fused polycyclic group containing 6 to 14 carbon atoms, preferably being 6-10 membered, for example, phenyl and naphthyl, most preferably phenyl. The aryl ring may be fused to a heteroaryl, heterocyclyl, or cycloalkyl ring, where the ring attached to the parent structure is the aryl ring, and its non-limiting examples include: 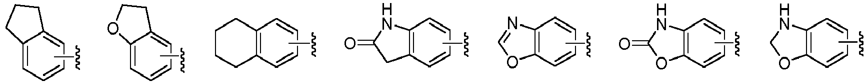 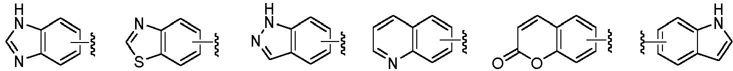 and the like.

"Heteroaryl or heteroaromatic ring" refers to a heteroaromatic system comprising 5 to 14 ring atoms, of which 1 to 4 ring atoms are selected from heteroatoms including oxygen, sulfur, and nitrogen. Heteroaryl is preferably 5-10 membered, more preferably heteroaryl is 5-6 membered, for example furyl, thienyl, pyridinyl, pyrrolyl, pyrimidinyl, pyrazinyl, pyrazolyl, imidazolyl, tetrazolyl, oxazolyl, isoxazolyl, thiazolyl, isothiazolyl, quinolinyl, isoquinolyl, indolyl, isoindolyl, and the like. The heteroaryl ring may be fused to an aryl, heterocyclyl, or cycloalkyl ring, where the ring attached to the parent structure is the heteroaryl ring, and its non-limiting examples include: 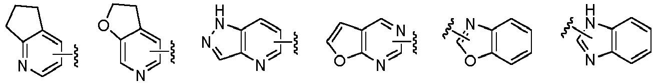 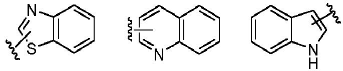 and the like.

"Halogen" refers to fluorine, chlorine, bromine or iodine.

"Cyano" refers to CN.

"Optionally" means that the subsequently described event or circumstance can but need not occur and that the expression includes instances where the event or circumstance occurs or does not occur. For example, a "heterocyclic group optionally substituted with an alkyl group" means that an alkyl group may but need not be present, and the expression includes cases where the heterocyclic group is substituted with an alkyl group and cases where the heterocyclic group is not substituted with an alkyl group.

"Substitution" refers to one or more hydrogen atoms, preferably 5, more preferably 1 to 3 hydrogen atoms in a group are independently substituted with a corresponding number of substituents. It goes without saying that substituents are only in their possible chemical positions and that a person skilled in the art can determine (by experiment or theory) possible or impossible substitutions without undue effort. For example, an amino or hydroxyl group with free hydrogen may be unstable when bonded to a carbon atom with an unsaturated (e.g., ethylenic) bond. The substituents include but are not limited to, halogen, cyano, nitro, oxo, -SFs, C₁₋₄alkyl, C₃₋₇cycloalkyl, 4-7 membered heterocyclyl, phenyl, 5-6 membered heteroaryl and the like.

"Isomer" refers to compounds that have the same molecular formula but differ in the nature or sequence of bonding of their atoms or the spatial arrangement of their atoms. Isomers that differ in the spatial arrangement of their atoms are referred to as "stereoisomers". Stereoisomers include optical isomers, geometric isomers, and conformational isomers.

The compounds of the present invention may exist in the form of optical isomers. Optical isomers include enantiomers and diastereoisomers. Enantiomers are two stereoisomers that are mirror images of each other but are not superimposable. A racemic mixture or racemate refers to a mixture of equal amounts of the left- and right-handed enantiomers of a chiral molecule. Diastereoisomers mean that two stereoisomers are not mirror images of each other and are not superimposable. When the optical isomer is a single isomer and its absolute configuration is determined, according to the configuration of the substituent on the chiral carbon atom, it is the absolute configuration of "R" or "S"; when the absolute configuration of the optical isomer is not determined, it is (+) or (-) according to the measured optical rotation. Methods of preparing and separating optical isomers are known in the art.

The compounds of the present invention may also exist as geometric isomers. The present invention contemplates various geometric isomers and mixtures thereof resulting from the distribution of substituents around the carbon-carbon double bond, the carbon-nitrogen double bond, the cycloalkyl, or the heterocyclic group. Substituents around the carbon-carbon double bond or the carbon-nitrogen bond are assigned Z or E configurations, and substituents around the cycloalkyl or the heterocycle are assigned cis or trans configurations.

The compounds of the present invention may also exhibit tautomerism, e.g. keto-enol tautomerism.

It should be understood that the present invention includes any tautomeric or stereoisomeric form and mixtures thereof, and is not limited to any one tautomeric or stereoisomeric form used in the nomenclature or chemical structural formula of the compound.

"Isotopes" refer to all isotopes of atoms occurring in the compounds of the present invention. Isotopes include those atoms having the same atomic number but different mass numbers. Examples of isotopes suitable for incorporation into compounds of the present invention are hydrogen, carbon, nitrogen, oxygen, phosphorus, sulfur, fluorine, and chlorine, for example, but not limited to, ²H (D), ³H, ¹³C, ¹⁴C, ¹⁵N, ¹⁷O, ¹⁸O, ³¹P, ³²P, ³⁵S,¹⁸F and ³⁶Cl. Isotopically-labeled compounds of the present invention can generally be prepared by conventional techniques known to those skilled in the art or by methods analogous to those described in the appended Examples using appropriate isotopically-labeled reagents in place of non-isotopically-labeled reagents. Such compounds have various potential uses, for example as standards and reagents in assays of biological activity. In the case of stable isotopes, such compounds have the potential to advantageously alter biological, pharmacological, or pharmacokinetic properties. Deuterium (D) is the preferred isotope of the present invention, e.g. hydrogen in methyl, methylene or methine may be replaced by deuterium.

The compounds of the present invention may be administered in the form of prodrugs. "Prodrug" refers to a derivative that is converted into a biologically active compound of the present invention under physiological conditions in vivo, such as by oxidation, reduction, hydrolysis, or the like (each of which is carried out using an enzyme or without the participation of an enzyme). Examples of prodrugs include the following compounds in which the amino group in the compound of the present invention is acylated, alkylated or phosphorylated, for example, eicosanoylamino, alanylamino, pivaloyloxymethylamino, or in which the hydroxyl group is acylated, alkylated, phosphorylated or converted to borate, e.g. acetoxy, palmitoyloxy, pivaloyloxy, succinyloxy, fumaryloxy, alanyloxy, or in which the carboxyl group is esterified or amidated, or in which the sulfhydryl group forms a disulfide bridge with a carrier molecule, such as a peptide, that selectively delivers the drug to the target and/or to the cytosol of the cell. These compounds can be prepared from the compounds of the present invention according to known methods.

"Pharmaceutically acceptable salt" refers to salts prepared from pharmaceutically acceptable bases or acids, including inorganic bases or acids and organic bases or acids. In case the compounds of the present invention contain one or more acidic or basic groups, the present invention also includes their corresponding pharmaceutically acceptable salts. Compounds according to the present invention which contain acidic groups can thus exist in salt form and can be used according to the present invention, for example as alkali metal salts, alkaline earth metal salts, or ammonium salts. More specific examples of such salts include sodium salt, potassium salt, calcium salt, magnesium salt, or salts formed with ammonia or organic amines such as ethylamine, ethanolamine, triethanolamine, or amino acids. The compounds according to the present invention which contain basic groups can exist in the form of salts and can be used according to the present invention in the form of their addition salts with inorganic or organic acids. Examples of suitable acids include hydrochloric acid, hydrobromic acid, phosphoric acid, sulfuric acid, nitric acid, methanesulfonic acid, p-toluenesulfonic acid, naphthalene disulfonic acid, oxalic acid, acetic acid, tartaric acid, lactic acid, salicylic acid, benzoic acid, formic acid, propanoic acid, pivalic acid, malonic acid, succinic acid, pimelic acid, fumaric acid, maleic acid, malic acid, sulfamic acid, phenylpropanoic acid, gluconic acid, ascorbic acid, isonicotinic acid, citric acid, adipic acid and other acids known to those skilled in the art. If the compounds according to the present invention simultaneously contain acidic and basic groups in the molecule, the present invention also includes, in addition to the salt forms mentioned, inner salts or betaines. The individual salts can be obtained by conventional methods known to those skilled in the art, for example by contacting the compounds of the present invention with organic or inorganic acids or bases in a solvent or dispersant or by anion exchange or cation exchange with other salts.

"Pharmaceutical composition" refers to compositions containing one or more compounds of the present invention or pharmaceutically acceptable salts, stable isotope derivatives, isomers, prodrugs or mixtures thereof, and other components such as pharmaceutically acceptable carriers and adjuvants. The purpose of the pharmaceutical composition is to promote the administration to the organism, facilitate the absorption of the active ingredient, and thus exert biological activity.

Therefore, when referring to "a compound", "a compound of the present invention" or "the compound of the present invention" in this application, all forms of said compounds are included, e.g. pharmaceutically acceptable salts, stable isotope derivatives, isomers, prodrugs or mixtures thereof.

As used herein, "cancer/tumor" includes but is not limited to digestive tract/gastrointestinal tract cancer, colon cancer, liver cancer, breast cancer, ovarian cancer, prostate cancer, head and neck cancer, skin cancer, lymphoma, leukemia (including acute myeloid leukemia and chronic myelogenous leukemia), kidney cancer, lung cancer, muscle cancer, bone cancer, bladder cancer, brain cancer, melanoma, multiple myeloma, vascular proliferation-related diseases/tumors.

As used herein, "inflammatory disease or autoimmune disease" includes, but is not limited to, arthritis, Hashimoto's thyroiditis, autoimmune hemolytic anemia, autoimmune atrophic gastritis with pernicious anemia, autoimmune encephalomyelitis, autoimmune orchitis, Goodpasture's disease, autoimmune thrombocytopenia, sympathetic ophthalmia, myasthenia gravis, Graves' disease, primary biliary cirrhosis, hepatitis, primary sclerotic cholangitis, chronic aggressive hepatitis, non-alcoholic fatty liver disease, non-alcoholic fatty hepatitis, ulcerative colitis, membranous glomerulopathy, systemic lupus erythematosus, rheumatoid arthritis, psoriatic arthritis, Sjogren syndrome, Reiter syndrome, polymyositis, dermatomyositis, Type I interferon diseases, including Aicardi-Goutières syndrome and other systemic sclerosis that overexpress type I interferon, Mendelian diseases, polyarteritis nodosa, multiple sclerosis, relapsing-remitting multiple sclerosis, primary progressive multiple sclerosis, secondary progressive multiple sclerosis, bullous pemphigoid; enteritis includes but is not limited to Crohn's disease, ulcerative colitis, inflammatory bowel disease, celiac disease, proctitis, eosinophilic gastroenteritis, and mastocytosis; skin diseases include but are not limited to atopic dermatitis, eczema, psoriasis, scleroderma, pruritus or other itching symptoms, vitiligo, and hair loss; O-cell (humoral) or T-cell based autoimmune diseases, including Cogan's syndrome, ankylosing spondylitis, Wegener's granulomatosis, autoimmune alopecia, type I or juvenile diabetes, thyroiditis, and the like;anaphylaxis such as allergic dermatitis, summer eczema, itchy horseshoes, spasm, inflammatory airway diseases, repeated airway obstruction, airway hyperresponsiveness, chronic obstructive pulmonary disease, and the like; asthma and other obstructive airway diseases, including but not limited to chronic or excessive asthma, delayed asthma, bronchitis, bronchial asthma, allergic asthma, endogenous asthma, exogenous asthma, and dusty asthma.

As used herein, a "therapeutically effective amount" refers to the inclusion of an amount of the compound of the present invention that is effective in treating or preventing the disease.

As used herein, "patient" refers to a mammal, especially a human.

The present invention provides a compound represented by general formula (I) as a JAK inhibitor, or a pharmaceutically acceptable salt thereof, a stable isotope derivative thereof, an isomer thereof, or a prodrug thereof: 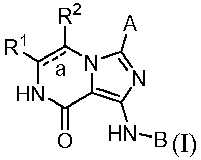 wherein:
bond a is a single bond or a double bond;
R¹ and R² are each independently selected from H, D, CN, C₁₋₆alkyl, and C₃₋₆cycloalkyl, wherein one or more hydrogens of the alkyl are optionally substituted by D or fluorine;
A is C₃₋₁₀cycloalkyl, 4-10 membered heterocyclyl, C₆₋₁₀aryl or 5-10 membered heteroaryl, wherein one or more hydrogens of the cycloalkyl, heterocyclyl, phenyl, and heteroaryl are optionally substituted by a substituent selected from D, halogen, cyano, -OR^{a}, -NR^{a}R^{b}, -C(O)R^{a}, -C(O)NR^{a}R^{b}, -S(O)₂R^{a}, - P(O)(CH₃)₂, C₁₋₆alkyl, C₃₋₆cycloalkyl, 4-8 membered heterocyclyl and 5-6 membered heteroaryl;
B is phenyl or 5-6 membered heteroaryl, wherein one or more hydrogens of the phenyl and heteroaryl are optionally substituted by a substituent selected from D, halogen, cyano, -OR^{a}, -NR^{a}R^{b}, -COOR^{a}, - C(O)R^{a}, -NR^{a}C(O)R^{b}, -C(O)NR^{a}R^{b}, -S(O)₂R^{a}, -S(O)₂NR^{a}R^{b}, -S(O)(NR^{a})R^{b}, -P(O)(CH₃)₂ and R¹¹;
R¹¹ is C₁₋₆alkyl, C₃₋₆cycloalkyl, 4-8 membered heterocyclyl or 5-6 membered heteroaryl, wherein one or more hydrogens of the alkyl, cycloalkyl, heterocyclyl and heteroaryl are optionally substituted by a substituent selected from D, halogen, CN, -OH, -NH₂, C₁₋₆alkyl, -OC₁₋₆alkyl, -COOR^{a}, -C(O)R^{a} and - C(O)NR^{a}R^{b};
R^{a} and R^{b} are each independently selected from H, C₁₋₆alkyl, C₃₋₆cycloalkyl, and 4-8 membered heterocyclyl, wherein one or more hydrogens of the alkyl, cycloalkyl, and heterocyclyl are optionally substituted by D, halogen or C₁₋₆alkyl.

In a preferable embodiment, R¹ and R² are both H.

In a preferable embodiment, A is C₃₋₈cycloalkyl, 4-8 membered heterocyclyl, phenyl or 5-6 membered heteroaryl, wherein one or more hydrogens of the cycloalkyl, heterocyclyl, phenyl and heteroaryl are optionally substituted by a substituent selected from halogen, -C(O)R^{a}, C₁₋₆alkyl and C₃₋₆cycloalkyl.

In a more preferable embodiment, A is phenyl, wherein one or more hydrogens of the phenyl are optionally substituted by halogen.

In an embodiment, B is phenyl or 5-6 membered heteroaryl, wherein one or more hydrogens of the phenyl and heteroaryl are optionally substituted by a substituent selected from halogen, -COOR^{a}, - C(O)R^{a}, -C(O)NR^{a}R^{b}, -S(O)₂R^{a}, C₁₋₆alkyl, C₃₋₆cycloalkyl, and 4-8 membered heterocyclyl containing N, S and/or O heteroatom(s), one or more hydrogens of the alkyl, cycloalkyl and heterocyclyl are optionally further substituted by a substituent selected from C₁₋₆alkyl, -C(O)R^{a} and -C(O)NR^{a}R^{b}.

In an embodiment, R^{a} and R^{b} are each independently selected from H, C₁₋₆alkyl, C₃₋₆cycloalkyl, and 4-6 membered heterocyclyl containing N, S, and/or O heteroatom(s), wherein one or more hydrogens of the alkyl, cycloalkyl, and heterocyclyl are optionally substituted by C₁₋₆alkyl.

In some embodiments, the compound of general formula (I) is represented by general formula (II): 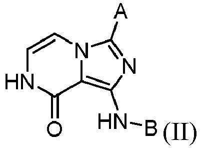 wherein:
A is C₃₋₈cycloalkyl, 4-8 membered heterocyclyl, phenyl or 5-6 membered heteroaryl, wherein one or more hydrogens of the cycloalkyl, heterocyclyl, phenyl and heteroaryl are optionally substituted by a substituent selected from halogen, -C(O)R^{a}, C₁₋₆alkyl and C₃₋₆cycloalkyl;
In a preferable embodiment, A is phenyl, wherein one or more hydrogens of the phenyl are optionally substituted by halogen;
B is phenyl or 5-6 membered heteroaryl, wherein one or more hydrogens of the phenyl and heteroaryl are optionally substituted by a substituent selected from halogen, -COOR^{a}, -C(O)R^{a}, -C(O)NR^{a}R^{b}, - S(O)₂R^{a}, C₁₋₆alkyl, C₃₋₆cycloalkyl, and 4-8 membered heterocyclyl containing N, S and/or O heteroatom(s), one or more hydrogens of the alkyl, cycloalkyl and heterocyclyl are optionally further substituted by a substituent selected from C₁₋₆alkyl, -C(O)R^{a} and -C(O)NR^{a}R^{b};
R^{a} and R^{b} are each independently selected from H, C₁₋₆alkyl, C₃₋₆cycloalkyl, and 4-6 membered heterocyclyl containing N, S, and/or O heteroatom(s), wherein one or more hydrogens of the alkyl, cycloalkyl, and heterocyclyl are optionally substituted by C₁₋₆alkyl.

In other embodiments, the compound of general formula (I) is represented by general formula (III): 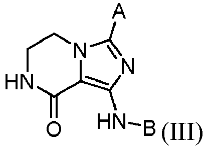 wherein:
A is phenyl, wherein one or more hydrogens of the phenyl are optionally substituted by halogen;
B is phenyl or 5-6 membered heteroaryl, wherein one or more hydrogens of the phenyl and heteroaryl are optionally substituted by a substituent selected from halogen, -COOR^{a}, -C(O)R^{a}, -C(O)NR^{a}R^{b}, - S(O)₂R^{a}, C₁₋₆alkyl, C₃₋₆cycloalkyl, and 4-8 membered heterocyclyl containing N, S and/or O heteroatom(s), one or more hydrogens of the alkyl, cycloalkyl and heterocyclyl are optionally further substituted by a substituent selected from C₁₋₆alkyl, -C(O)R^{a} and -C(O)NR^{a}R^{b};
R^{a} and R^{b} are each independently selected from H, C₁₋₆alkyl, C₃₋₆cycloalkyl, and 4-6 membered heterocyclyl containing N, S, and/or O heteroatom(s), wherein one or more hydrogens of the alkyl, cycloalkyl, and heterocyclyl are optionally substituted by C₁₋₆alkyl.

The present invention also relates to the following compounds 1-40, or pharmaceutically acceptable salts thereof, stable isotope derivatives thereof, isomers thereof or prodrugs thereof, or mixtures thereof.

| Compound No. | Compound structures and names |
|---|---|
| 1. | 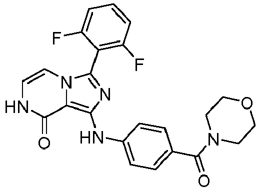 |
| | 3-(2,6-difluorophenyl)-1-((4-(morpholine-4-carbonyl)phenyl)amino)imidazo[1,5-a]pyrazin-8(7H)-one |
| 2. | 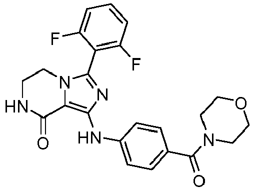 |
| | 3-(2,6-difluorophenyl)-1-((4-(morpholine-4-carbonyl)phenyl)amino)-6,7-dihydroimidazo[1,5-a]pyrazin-8(5H)-one |
| 3. | 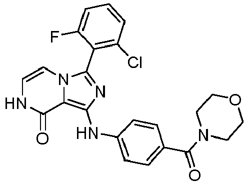 |
| | 3-(2-chloro-6-fluorophenyl)-1-((4-(morpholine-4-carbonyl)phenyl)amino)imidazo[1,5-a]pyrazin-8(7H)-one |
| 4. | 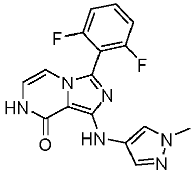 |
| | 3-(2,6-difluorophenyl)-1-((1-methyl-1H-pyrazol-4-yl)amino)imidazo[1,5-a]pyrazin-8(7H)-one |
| 5. | 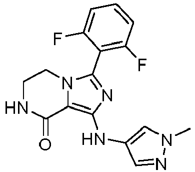 |
| | 3-(2,6-difluorophenyl)-1-((1-methyl-1H-pyrazol-4-yl)amino)-6,7-dihydroimidazo[1,5-a]pyrazin-8(5H)-one |
| 6. | 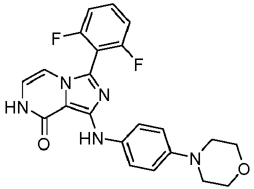 |
| | 3-(2,6-difluorophenyl)-1-((4-morpholinophenyl)amino)imidazo[1,5-a]pyrazin-8(7H)-one |
| 7. | 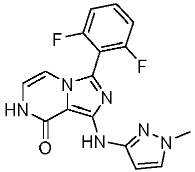 |
| | 3-(2,6-difluorophenyl)-1-((1-methyl-1H-pyrazol-3-yl)amino)imidazo[1,5-a]pyrazin-8(7H)-one |
| 8. | 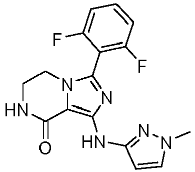 |
| | 3-(2,6-difluorophenyl)-1-((1-methyl-1H-pyrazol-3-yl)amino)-6,7-dihydroimidazo[1,5-a]pyrazin-8(5H)-one |
| 9. | 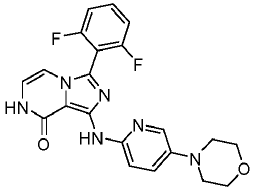 |
| | 3-(2,6-difluorophenyl)-1-((5-morpholinopyridin-2-yl)amino)imidazo[1,5-a]pyrazin-8(7H)-one |
| 10. | 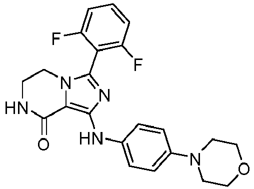 |
| | 3-(2,6-difluorophenyl)-1-((4-morpholinophenyl)amino)-6,7-dihydroimidazo[1,5-a]pyrazin-8(5H)-one |
| 11. | 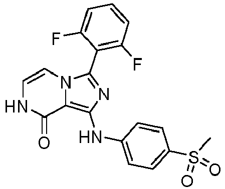 |
| | 3-(2,6-difluorophenyl)-1-((4-(methylsulfonyl)phenyl)amino)imidazo[1,5-a]pyrazin-8(7H)-one |
| 12. | 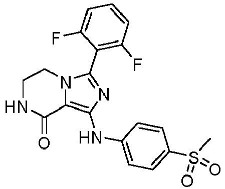 |
| | 3-(2,6-difluorophenyl)-1-((4-(methylsulfonyl)phenyl)amino)-6,7-dihydroimidazo[1,5-a]pyrazin-8(5H)-one |
| 13. | 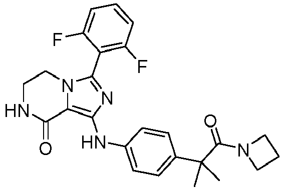 |
| | 1-((4-(1 -(azetidin-1 -yl)-2-methyl-1 -oxopropan-2-yl)phenyl)amino)-3 -(2,6-difluorophenyl)-6,7-dihydroimidazo[1,5-a]pyrazin-8(5H)-one |
| 14. | 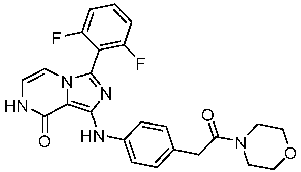 |
| | 3-(2,6-difluorophenyl)-1-((4-(2-morpholino-2-oxoethyl)phenyl)amino)imidazo[1,5-a]pyrazin-8(7H)-one |
| 15. | 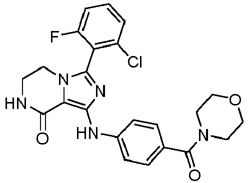 |
| | 3-(2-chloro-6-fluorophenyl)-1-((4-(morpholine-4-carbonyl)phenyl)amino)-6,7-dihydroimidazo[1,5-a]pyrazin-8(5H)-one |
| 16. | 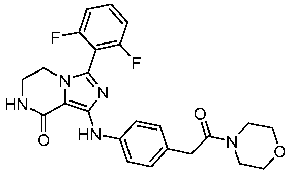 |
| | 3-(2,6-difluorophenyl)-1-((4-(2-morpholino-2-oxoethyl)phenyl)amino)-6,7-dihydroimidazo[1,5-a]pyrazin-8(5H)-one |
| 17. | 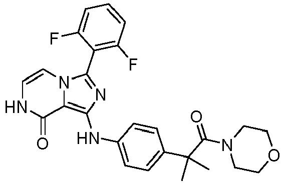 |
| | 3-(2,6-difluorophenyl)-1-((4-(2-methyl-1-morpholino-1-oxopropan-2-yl)phenyl)amino)imidazo[1,5-a]pyrazin-8(7H)-one |
| 18. | 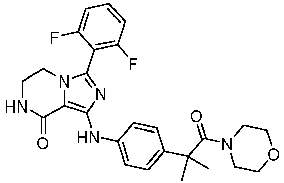 |
| | 3-(2,6-difluorophenyl)-1-((4-(2-methyl-1-morpholino-1-oxopropan-2-yl)phenyl)amino)-6,7-dihydroimidazo[1,5-a]pyrazin-8(5H)-one |
| 19. | 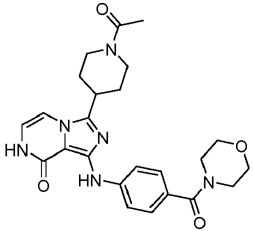 |
| | 3 -(1 -acetylpiperidin-4-yl)- 1 -((4-(morpholine-4-carbonyl)phenyl)amino)imidazo[1,5-a]pyrazin-8(7H)-one |
| 20. | 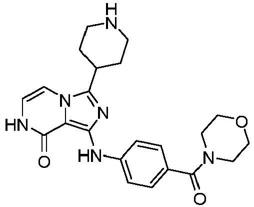 |
| | 1-((4-(morpholine-4-carbonyl)phenyl)amino)-3-(piperidin-4-yl)imidazo[1,5-a]pyrazin-8(7H)-one |
| 21. | 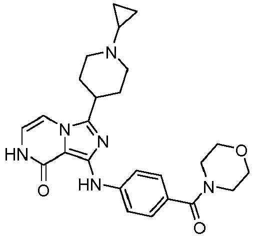 |
| | 3 -(1 -cyclopropylpiperidin-4-yl)- 1 -((4-(morpholine-4-carbonyl)phenyl)amino)imidazo[1,5-a]pyrazin-8(7H)-one |
| 22. | 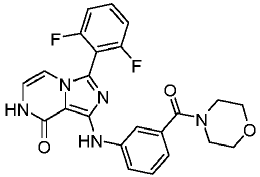 |
| | 3-(2,6-difluorophenyl)-1-((3 -(morpholine-4-carbonyl)phenyl)amino)imidazo[1,5-a]pyrazin-8(7H)-one |
| 23. | 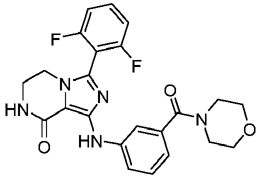 |
| | 3-(2,6-difluorophenyl)-1-((3-(morpholine-4-carbonyl)phenyl)amino)-6,7-dihydroimidazo[1,5-a]pyrazin-8(5H)-one |
| 24. | 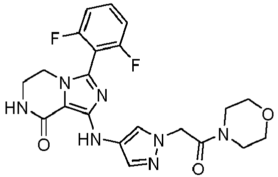 |
| | 3-(2,6-difluorophenyl)-1-((1-(2-morpholino-2-oxoethyl)-1H-pyrazol-4-yl)amino)-6,7-dihydroimidazo[1,5-a]pyrazin-8(5H)-one |
| 25. | 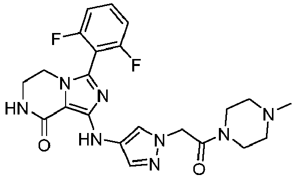 |
| | 3-(2,6-difluorophenyl)-1-((1-(2-(4-methylpiperazin-1-yl)-2-oxoethyl)-1H-pyrazol-4-yl)amino)-6,7-dihydroimidazo[1,5-a]pyrazin-8(5H)-one |
| 26. | 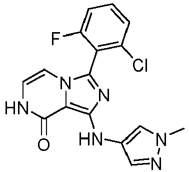 |
| | 3-(2-chloro-6-fluorophenyl)-1-((1-methyl-1H-pyrazol-4-yl)amino)imidazo[1,5-a]pyrazin-8(7H)-one |
| 27. | 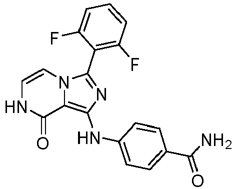 |
| | 4-((3-(2,6-difluorophenyl)-8-oxo-7,8-dihydroimidazo[1,5-a]pyrazin-1-yl)amino)benzamide |
| 28. | 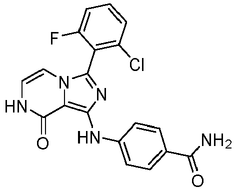 |
| | 4-((3-(2-chloro-6-fluorophenyl)-8-oxo-7,8-dihydroimidazo[1,5-a]pyrazin-1-yl)amino)benzamide |
| 29. | 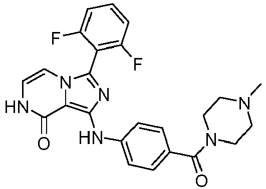 |
| | 3 -(2,6-difluorophenyl)-1 -((4-(4-methylpiperazine-1 - carbonyl)phenyl)amino)imidazo[1,5-a]pyrazin-8(7H)-one |
| 30. | 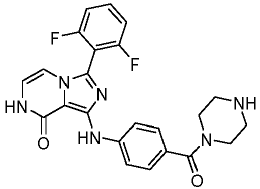 |
| | 3-(2,6-difluorophenyl)-1-((4-(piperazine- 1 - carbonyl)phenyl)amino)imidazo[1,5-a]pyrazin-8(7H)-one |
| 31. | 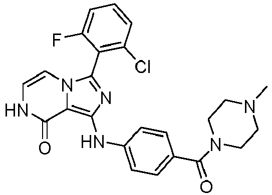 |
| | 3 -(2-chloro-6-fluorophenyl)-1 -((4-(4-methylpiperazine-1 - carbonyl)phenyl)amino)imidazo[1,5-a]pyrazin-8(7H)-one |
| 32. | 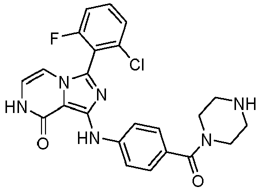 |
| | 3-(2-chloro-6-fluorophenyl)-1-((4-(piperazine- 1 - carbonyl)phenyl)amino)imidazo[1,5-a]pyrazin-8(7H)-one |
| 33. | 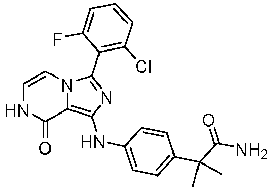 |
| | 2-(4-((3-(2-chloro-6-fluorophenyl)-8-oxo-7,8-dihydroimidazo[1,5-a]pyrazin-1-yl)amino)phenyl)-2-methylpropanamide |
| 34. | 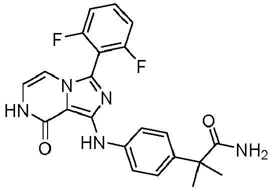 |
| | 2-(4-((3-(2,6-difluorophenyl)-8-oxo-7,8-dihydroimidazo[1,5-a]pyrazin-1-yl)amino)phenyl)-2-methylpropanamide |
| 35. | 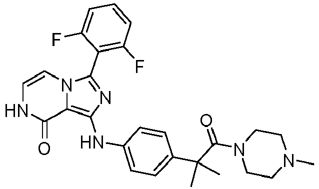 |
| | 3 -(2,6-difluorophenyl)-1 -((4-(2-methyl-1 -(4-methylpiperazin-1 -yl)-1 - oxopropan-2-yl)phenyl)amino)imidazo[1,5-a]pyrazin-8(7H)-one |
| 36. | 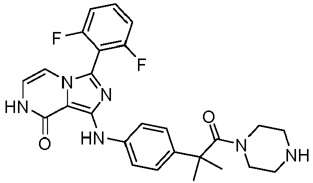 |
| | 3 -(2,6-difluorophenyl)-1 -((4-(2-methyl-1 -oxo-1 -(piperazin-1 -yl)propan-2-yl)phenyl)amino)imidazo[1,5-a]pyrazin-8(7H)-one |
| 37. | 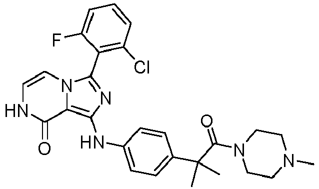 |
| | 3 -(2-chl oro-6-fluorophenyl)-1 -((4-(2-methyl-1 -(4-m ethylpip erazin-1 -yl)-1 - oxopropan-2-yl)phenyl)amino)imidazo[1,5-a]pyrazin-8(7H)-one |
| 38. | 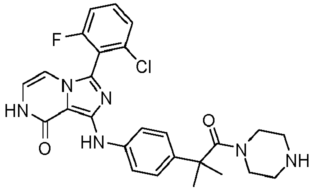 |
| | 3-(2-chloro-6-fluorophenyl)-1-((4-(2-methyl-1-oxo-1-(piperazin-1-yl)propan-2-yl)phenyl)amino)imidazo[1,5-a]pyrazin-8(7H)-one |
| 39. | 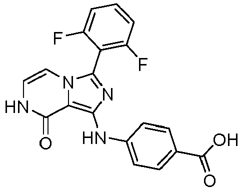 |
| | 4-((3-(2,6-difluorophenyl)-8-oxo-7,8-dihydroimidazo[1,5-a]pyrazin-1-yl)amino)benzoic acid |
| 40. | 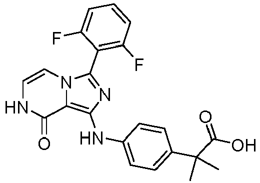 |
| | 2-(4-((3-(2,6-difluorophenyl)-8-oxo-7,8-dihydroimidazo[1,5-a]pyrazin-1-yl)amino)phenyl)-2-methylpropanoic acid |

The compounds of the present invention can effectively inhibit the activity of JAK, preferably with an IC₅₀ less than 100 nM, and more preferably with an IC₅₀ less than 10 nM.

The present invention also relates to a pharmaceutical composition comprising a compound represented by general formula (I) or a pharmaceutically acceptable salt thereof, a stable isotope derivative thereof, an isomer thereof and a prodrug thereof, and one or more pharmaceutically acceptable carriers or adjuvants.

Another aspect of the present invention provides a method for treating or preventing JAK-mediated diseases, wherein the method comprises administering a patient in need thereof a therapeutically effective amount of the compound represented by general formula (I) or a pharmaceutically acceptable salt thereof, a stable isotope derivative thereof, an isomer thereof, a prodrug thereof, or a mixture thereof, or a pharmaceutical composition containing the compound; the diseases include but are not limited to inflammatory diseases including enteritis, autoimmune diseases, cancer, and the like, especially inflammatory bowel disease, dermatitis, eczema, rheumatoid arthritis, systemic lupus erythematosus, psoriasis, alopecia areata, and the like.

According to the present invention, the drug may be in any pharmaceutical dosage form, including but not limited to tablets, capsules, solutions, freeze-dried preparations, and injections.

The pharmaceutical preparations of the present invention may be administered in dosage unit forms containing a predetermined amount of active ingredient per dosage unit. Such units may contain, for example, 0.5 mg to 1 g, preferably 1 mg to 700 mg, particularly preferably 5 mg to 500 mg, of the compound of the present invention depending on the condition to be treated, the method of administration and the age, weight, and condition of the patient. Furthermore, pharmaceutical preparations of this type can be prepared using methods known in the field of pharmacy, such as mixing the active ingredient with one or more adjuvants and/or excipients.

The pharmaceutical preparations of the present invention may be adapted for administration by any desired suitable method, such as oral (including buccal or sublingual), rectal, nasal, topical (including buccal, sublingual or transdermal), vaginal or parenteral (including subcutaneous, intramuscular, intravenous or intradermal) administration.

The present invention also provides methods for preparing the compounds. The preparation of the compound represented by general formula (I) of the present invention can be accomplished through the following exemplary methods and examples, but these methods and examples should not be considered as limiting the scope of the present invention in any way. The compounds described in the present invention can also be synthesized by synthetic techniques known to those skilled in the art, or a combination of methods known in the art and methods described in the present invention can be used. The products obtained in each reaction step are obtained by separation techniques known in the art, including but not limited to extraction, filtering, distillation, crystallization, chromatographic separation, and the like. The starting materials and chemical reagents required for the synthesis can be routinely synthesized according to the literature (available on SciFinder) or purchased.

### Synthesis method

The heterocyclic compound of general formula (I) of the present invention can be synthesized according to the following route: 1) a carboxylic acid A1 and an amine A2 are subjected to condensation to produce A3; 2) A3 is dehydrated and cyclized under an acidic condition to produce A4; 3) A4 is brominated with NBS to produce A5; 4) A5 reacts with sodium methoxide to produce A6; 5) A6 and an amine B-NH2 are subjected to a Bulkwald coupling reaction to produce A7; 6) A7 is unprotected to produce A8. Functional groups FG1 and FG2 of Some A7s and A8s can be further derivatized to produce various target compounds, for example, FG1 contains a protected amine and is deprotected to produce an amine, which is further subjected to amidation or reductive amination to produce an amide and a substituted amine; again for example, an ester in FG2 is hydrolyzed with a base (e.g. LiOH) to form an acid, which is further amidated to produce an amide; the like.

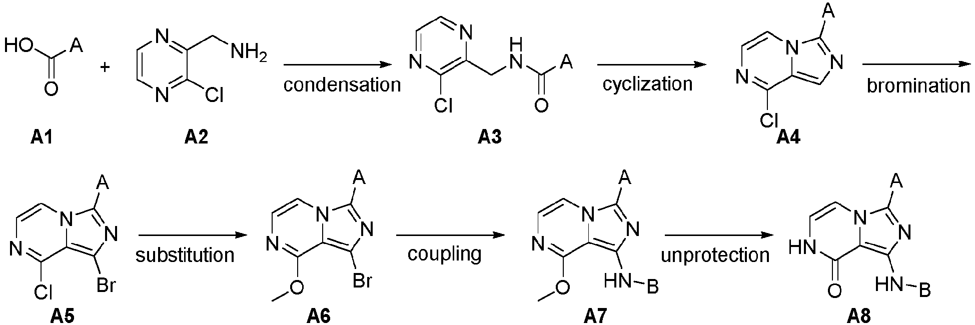

The heterocyclic compound of the general formula (I) of the present invention can also be synthesized according to the following route: 1) A8 is hydrogenated to produce B1. Functional groups FG1 and FG2 of some B1s can be further derivatized to produce various target compounds, e.g. an ester in FG2 is hydrolyzed with a base (such as LiOH) to form an acid, which is further amidated to produce an amide and the like.

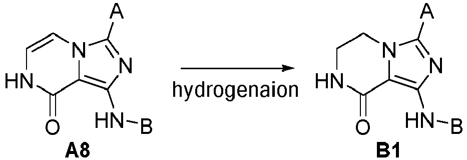

### Examples

The starting materials of the present invention could be synthesized according to methods known in the art or could be purchased from chemical companies such as ABCR GmbH&Co. KG, Acros Organics, Aldrich Chemical Company, Accela ChemBio Inc., and Beijing OUHE Technology Co. Ltd.

The structures of the compounds of the present invention were determined by nuclear magnetic resonance (NMR) and/or mass spectroscopy (MS). The NMR determination was performed with a Bruker ASCEND-400 nuclear magnetic analyzer, by using solvents such as deuterated dimethyl sulfoxide (DMSO-*d₆*), deuterated chloroform (CDCl₃), or deuterated methanol (CD₃OD), using tetramethylsilane (TMS) as the internal standard, and giving chemical shifts in units of 10⁻⁶ (ppm). The MS determination was performed with an Agilent SQD (ESI) mass spectrometer (Agilent 6120).

The HPLC determination was performed with Agilent 1260 DAD high-pressure liquid chromatograph (Poroshell 120 EC-C18, 50×3.0 mm, 2.7 µm chromatographic column) or Waters Arc high-pressure liquid chromatograph (Sunfirc C18, 150×4.6 mm, 5 µm chromatographic column).

Unless otherwise specified in the examples, the reaction temperature was room temperature (20-30°C).

Unless otherwise specified in the examples, the reactions were carried out under an argon atmosphere or a nitrogen atmosphere. Argon atmosphere or nitrogen atmosphere meant that the reaction bottle was connected to an argon or nitrogen balloon with a volume of about 1 L.

Hydrogen atmosphere meant that the reaction bottle was connected to a hydrogen balloon with a volume of about 1 L after being vacuumed and then filled with hydrogen (repeatedly 3 times).

A CEM Discover-SP type microwave reactor was used in the microwave reaction.

The reaction progress in Examples was monitored with an LC/MS chromatography (1260/6120) of Agilent, or thin-layer chromatography (TLC) using a silica gel plate having a thickness of 0.15 to 0.2 mm (Qingdao Haiyang GF254).

The purification of the compound was performed with column chromatography using 200-300 mesh silica gel from Qingdao Haiyang or a thin-layer chromatography using a GF254 silica gel plate from Qingdao Haiyang having a thickness of 0.4 to 0.5 mm.

The developing solvent system for column chromatography or thin layer chromatography usually included a) dichloromethane and methanol system, b) petroleum ether and ethyl acetate system, or those as shown in the Examples. The volume ratio of solvents was adjusted according to the polarity of the compound, and could also be further adjusted by adding a small amount of triethylamine or other acidic or basic reagents.

The purification of the compound was also performed with a mass spectrometer-guided automatic preparation system (mass spectrometer detector: SQD2) of Waters, and a reversed-phase high-pressure column (XBridge-C18, 19×150 mm, 5 µm) was eluted at a flow rate of 20 mL/min with an appropriate acetonitrile/water (containing 0.1% trifluoroacetic acid or formic acid, or 0.05% ammonia water) gradient according to the polarity of the compound. In a part of examples, 1 N diluted hydrochloric acid could be added after the purification with the automatic preparation system, and then the solvent was removed under reduced pressure to produce a hydrochloride.

The abbreviation of DMF refers to N,N-dimethylformamide.

The abbreviation of TFA refers to trifluoroacetic acid.

The abbreviation of DIPEA refers to N,N-diisopropylethylamine.

The abbreviation of NBS refers to N-bromosuccinimide.

The abbreviation of HATU refers to 2-(7-aza-benzotriazole)-N,N,N',N'-tetramethyluronium hexafluorophosphate.

The abbreviation of XantPhos refers to 4,5-bis(diphenylphosphino)-9,9-dimethylxanthene.

The abbreviation of Pd₂(dba)₃ refers to tris(dibenzylideneacetone)dipalladium.

The abbreviation of Brettphos refers to 2-(dicyclohexylphosphino)-3,6-dimethoxy-2',4',6'-triisopropyl-1,1'-biphenyl.

### Example 1

### 1-bromo-3-(2,6-difluorophenyl)-8-methoxyimidazo[1,5-a]pyrazine (intermediate 1f)

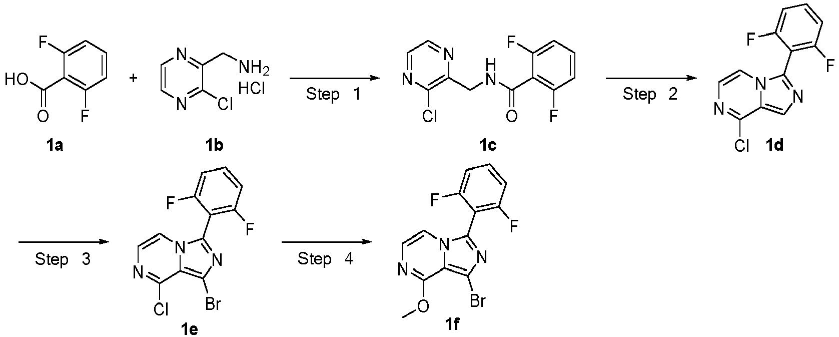

### Step 1

### N-((3-chloropyrazin-2-yl)methyl)-2,6-difluorobenzamide (1c)

2,6-difluorobenzoic acid 1a (5g, 31.6mmol) was dissolved in dichloromethane (100mL), and 3 drops of DMF were added. The resulting mixture was cooled to 0°C, and then oxalyl chloride (8g, 63.3mmol) was added dropwise. The resulting mixture was stirred at room temperature for 1 hour and then concentrated to dryness. The resulting residue was dissolved in dichloromethane (10mL) to produce a solution A. In another 250 mL round flask was added (3-chloropyrazin-2-yl)methylamine hydrochloride **1b** (5.7g, 31.6mmol), and then dichloromethane (100mL) and triethylamine (9.6g, 94.8mmol) were added. The resulting mixture was cooled to 0°C, and then the solution A was added dropwise. The mixture was stirred at room temperature for 1 hour and quenched with water (50mL). The separated organic phase was dried over anhydrous sodium sulfate, and filtered. The resulting filtrate was concentrated to dryness under a reduced pressure. The residue was purified with a silica gel column chromatography (ethyl acetate/dichloromethane=1/1) to produce the target product 1c (6.8g, 76%).
MS m/z (ESI): 284 [M+1]
¹H NMR (400 MHz, CDCl₃) δ 8.47 (d, *J=* 2.5 Hz, 1H), 8.39-8.30 (m, 1H), 7.44-7.37 (m, 2H), 7.03-6.90 (m, 2H), 4.93 (d, *J* = 4.5 Hz, 2H).

### Step 2

### 8-chloro-3-(2,6-difluorophenyl)imidazo[1,5-a]pyrazine (1d)

To a solution of **1c** (6.8g, 24mmol) in acetonitrile (80mL) was added phosphoryl chloride (18.4g, 120mmol). The mixture was heated to 90°C and stirred for 16 hours. The mixture was cooled to room temperature, and then phosphoryl chloride (18.4g, 120mmol) was added. The resulting mixture was heated again to 90°C and stirred for 28 hours. The mixture was cooled to room temperature, and concentrated to dryness. Then a saturated sodium bicarbonate solution (50mL) was added and the resulting solution was extracted with dichloromethane (2×100mL). The combined organic phases were dried over anhydrous sodium sulfate, and filtered. The resulting filtrate was concentrated to dryness under a reduced pressure. The residue was purified with a silica gel column chromatography (ethyl acetate/dichloromethane=1/2) to produce the target product **1d** (5.9g, 92%).
MS m/z (ESI): 266 [M+1]
¹H NMR (400 MHz, CDCl₃) δ 8.10 (s, 1H), 7.60-7.50 (m, 2H), 7.44 (d, *J* = 5.0 Hz, 1H), 7.14 (t, *J* = 8.1 Hz, 2H).

### Step 3

### 1-bromo-8-chloro-3-(2,6-difluorophenyl)imidazo[1,5-a]pyrazine (1e)

To a solution of **1d** (880mg, 3.31mmol) in acetonitrile (40mL) was added NBS (590mg, 3.31mmol). The mixture was stirred at room temperature for 18 hours and then concentrated to dryness. The residue was dissolved in ethyl acetate (100mL), washed with water (2×50mL), and then dried over anhydrous sodium sulfate. After filtering, the resulting filtrate was concentrated to dryness under a reduced pressure. The residue was purified with a silica gel column chromatography (petroleum ether/ethyl acetate=2/1) to produce the target product **1e** (940mg, 82%).
MS m/z (ESI): 344 [M+1]
¹H NMR (400 MHz, CDCl₃) δ 7.57 (tt, *J* = 8.5, 6.3 Hz, 1H), 7.49-7.46 (m, 1H), 7.41 (d, *J* = 5.0 Hz, 1H), 7.13 (t, *J* = 8.1 Hz, 2H).

### Step 4

### 1-bromo-3-(2,6-difluorophenyl)-8-methoxyimidazo[1,5-a]pyrazine (1f)

**1e** (590mg, 1.71mmol) was dissolved in methanol (40mL). The resulting mixture was cooled to 0°C. A sodium methoxide solution (463mg, 2.57mmol, 30% of the solution in methanol) was added dropwise. The resulting mixture was stirred for 2 hours and then poured into a saturated ammonium chloride solution (100mL). The resulting mixture was extracted with ethyl acetate (3× 50mL). The combined organic phases were dried over anhydrous sodium sulfate, and filtered. The resulting filtrate was concentrated to dryness under a reduced pressure. The residue was purified with a silica gel column chromatography (petroleum ether/ethyl acetate=1/1) to produce the target product **1f** (570mg, 98%).
MS m/z (ESI): 340 [M+1]
¹H NMR (400 MHz, CDCl₃) δ 7.56-7.49 (m, 1H), 7.21 (dt, *J=* 5.0, 1.9 Hz, 1H), 7.17 (d, *J=* 5.1 Hz, 1H), 7.13-7.08 (m, 2H), 4.18 (s, 3H).

The intermediates in the following table were all prepared according to the experimental steps of Example 1 except that in step 1, different carboxylic acids were used to replace 2,6-difluorobenzoic acid **1a**.

| Intermediate No. | Intermediate structure | Compound replacing **1a** | MS m/z (ESI) |
|---|---|---|---|
| **3f** | 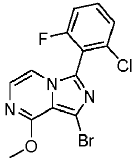 | 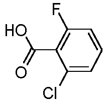 | 356 |
| **19a** | 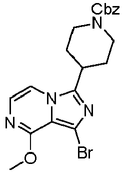 | 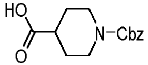 | 445 |

The nuclear magnetic resonance data of intermediates **3f** and **19a** were as follows:

| Intermediate | ¹H NMR |
|---|---|
| **1-bromo-3-(2-chloro-6-fluorophenyl)-8-methoxyimidazo[1,5-a]pyrazine (3f)** | ¹H NMR (400 MHz, CDCl₃) δ 7.48 (dd, *J* = 8.3, 5.9 Hz, 1H), 7.39 (dd, *J* = 8.2, 0.9 Hz, 1H), 7.21-7.16 (m, 1H), 7.15 (d, *J* = 5.1 Hz, 1H), 7.11 (dd, *J* = 5.0, 1.3 Hz, 1H), 4.17 (s, 3H). |
| **Benzyl 4-(1-bromo-8-methoxyimidazo[1,5-a]pyrazin-3-yl)piperidine-1-carboxylate (19a)** | ¹H NMR (400 MHz, CDCl₃) δ 7.40-7.29 (m, 6H), 7.09 (d, *J* = 5.1 Hz, 1H), 5.15 (s, 2H), 4.31 (d, *J* = 9.9 Hz, 2H), 4.10 (s, 3H), 3.12-2.94 (m, 3H), 1.95 (s, 4H). |

### Example 2

### 2-(4-aminophenyl)-2-methylpropanamide (intermediate 33c)

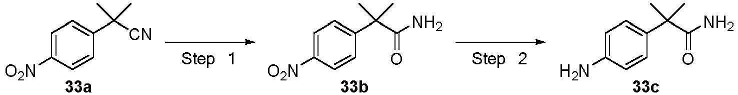

### Step 1

### 2-methyl-2-(4-nitrophenyl)propanamide (33b)

To a solution of 2-methyl-2-(4-nitrophenyl)propanenitrile **33a** (2.00g, 10.52mmol) in ethanol/water (20 mL, volume ratio 1/1) were added potassium carbonate (0.58g, 4.21mmol) and 30% aqueous hydrogen peroxide solution (35mL) at 0°C, and the resulting mixture was stirred at 0°C for 0.5 hours, then warmed up to room temperature and stirred for 16 hours. After the completion of the reaction, a saturated sodium sulfite solution (50mL) was added. The resulting mixture was stirred at room temperature for 0.5 hours, and then extracted with ethyl acetate (3×50mL). The combined organic phases were dried over anhydrous sodium sulfate, and filtered. The filtrate was concentrated to dryness under a reduced pressure. The residue was purified with a silica gel column chromatography (dichloromethane/methanol=20/1) to produce the target product **33b** (0.76g, 35%).

MS m/z (ESI): 209 [M+1]

### Step 2

### 2-(4-aminophenyl)-2-methylpropanamide (33c)

To a solution of **33b** (0.76g, 3.65mmol) in methanol (10mL) was added 20% palladium/carbon (0.15g). The mixture was stirred under an atmosphere of hydrogen gas for 12 hours, and then filtered. The filtrate was concentrated to dryness under a reduced pressure to produce the target product **33c** (0.65g, 100%).

MS m/z (ESI): 179 [M+1]

### Example 3

### Tert-butyl 4-(2-(4-aminophenyl)-2-methylpropanoyl)piperazine-1-carboxylate (intermediate 36d)

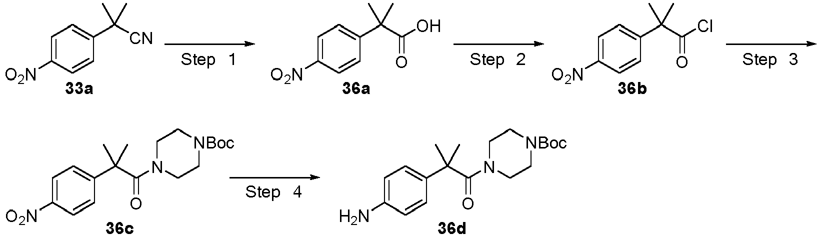

### Step 1

### 2-methyl-2-(4-nitrophenyl)propanoic acid (36a)

To a mixture of **33a** (5.00g, 26.29mmol) and water (25mL) was added a concentrated sulfuric acid (25mL) at 0°C, and then the resulting mixture was heated to 120°C and stirred for 12 hours. After cooling to room temperature, water (50mL) was added. The resulting mixture was adjusted to pH=8 with a saturated sodium bicarbonate solution, and then extracted with ethyl acetate (3× 50mL). The combined organic phases were dried over anhydrous sodium sulfate, and filtered. The filtrate was concentrated to dryness under a reduced pressure to produce the target product 36a (5.00g, 91%).

MS m/z (ESI): 210 [M+1]

### Step 2

### 2-methyl-2-(4-nitrophenyl)propanoylchloride (36b)

**36a** (5.00g, 23.90mmol) was added to thionyl chloride at 0°C. Then the resulting mixture was heated to 85°C and stirred for 12 hours. After cooling to room temperature, the mixture was concentrated to dryness to produce the target product **36b** (5.20g, 96%).

### Step 3

### Tert-butyl 4-(2-methyl-2-(4-nitrophenyl)propanoyl)piperazine-1-carboxylate

To a solution of *tert*-butyl piperazine-1-carboxylate (1.84g, 9.89mmol) in dichloromethane (20mL) was added triethylamine (2.00g, 19.77mmol) at room temperature. The resulting mixture was stirred for 10 minutes, and then cooled to 0°C. A solution of **36c** (1.50g, 6.59mmol) in dichloromethane (10mL) was added. The resulting mixture was stirred at room temperature for 2 hours, and then water (20mL) was added. The resulting mixture was extracted with dichloromethane (3×30mL). The combined organic phases were dried over anhydrous sodium sulfate, and filtered. The filtrate was concentrated to dryness. The residue was purified with a silica gel column chromatography (dichloromethane/methanol= 100/1) to produce the target product **36c** (1.50g, 60%).

MS m/z (ESI): 378 [M+1]

### Step 4

### Tert-butyl 4-(2-(4-aminophenyl)-2-methylpropanoyl)piperazine-1-carboxylate (36d)

To a solution of **36c** (1.50g, 3.97mmol) in methanol (250mL) was added 20% palladium/carbon (0.3 g). Then the resulting mixture was stirred under an atmosphere of hydrogen gas for 12 hours and filtered. The filtrate was concentrated to dryness to produce the target product **36d** (1.38g, 100%).

MS m/z (ESI): 348 [M+1]

Intermediate **35d** was synthesized according to the experimental steps of Example 3 except that in step *3, tert-butyl* piperazine-1-carboxylate was replaced with N-methylpiperidine.

| Intermediate No. | Intermediate structure | Compound replacing tert-butyl piperazine-1 -carboxylate | MS m/z (ESI) |
|---|---|---|---|
| **35d** | 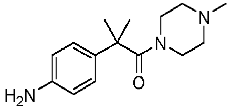 | 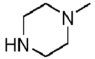 | 262 |

### Example 4

### 3-(2,6-difluorophenyl)-1-((4-(morpholine-4-carbonyl)phenyl)amino)imidazo[1,5-a]pyrazin-8(7H)-one (compound 1)

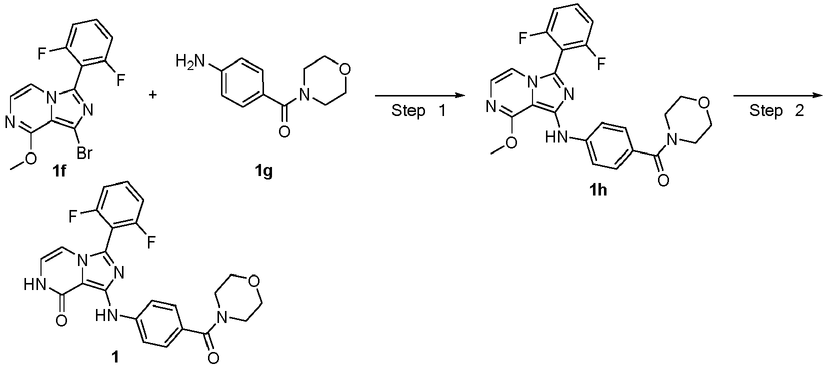

### Step 1

### (4-((3-(2,6-difluorophenyl)-8-methoxyimidazo[1,5-a]pyrazin-1-yl)amino)phenyl)(morpholino)methanone (1h)

To a mixture of **1f** (200mg, 0.59mmol), (4-aminophenyl)(morpholino)methanone **1g** (121mg, 0.59mmol) and 1,4-dioxane (2mL) was added sodium *tert*-butoxide (144mg, 1.5mmol), and then added XantPhos (69mg, 0.12mmol) and Pd₂(dba)₃ (55mg, 0.06mmol). The mixture was heated in a microwave reactor to 100°C and stirred for 1 hour. After cooling to room temperature, the reaction mixture was diluted with water (20mL) and then extracted with ethyl acetate (3×20mL). The combined organic phases were dried over anhydrous sodium sulfate, and filtered. The filtrate was concentrated to dryness under a reduced pressure. The residue was purified with a silica gel column chromatography (dichloromethane/methanol=20/1) to produce the target product **1h** (260mg, 95%).

MS m/z (ESI): 466 [M+1]

### Step 2

### 3-(2,6-difluorophenyl)-1-((4-(morpholine-4-carbonyl)phenyl)amino)imidazo[1,5-a]pyrazin-8(7H)-one (1)

To a solution of **1h** (260mg, 0.56mmol) in acetonitrile (10 mL) was added 6N hydrochloric acid (10mL). The resulting mixture was heated to 70°C and stirred for 1 hour. After cooling to room temperature, the mixture was concentrated to dryness. The residue was diluted with water (10mL), then adjusted to pH=8 with a saturated sodium bicarbonate solution, and extracted with ethyl acetate (2×50mL). The combined organic phases were dried over anhydrous sodium sulfate, and filtered. The filtrate was concentrated to dryness under a reduced pressure. The residue was purified with a silica gel column chromatography (dichloromethane/methanol= 20/1) to produce the target product 1 (solid, 150mg, 59%).
MS m/z (ESI): 452 [M+1]
¹H NMR (400 MHz, DMSO-*d₆*) δ 10.56 (d, *J* = 5.3 Hz, 1H), 8.42 (s, 1H), 7.78-7.63 (m, 3H), 7.43-7.27 (m, 4H), 6.84 (d, *J* = 5.3 Hz, 1H), 6.59 (t, *J* = 5.7 Hz, 1H), 3.59 (d, *J* = 4.5 Hz, 4H), 3.50 (s, 4H).

The compounds or intermediates in the following table were all prepared according to the experimental steps of Example 4 except that in step 1, different compounds were respectively used to replace **1f** and **1g**.

| No. | Compound structure | Compound replacing 1f | Compound replacing 1g | MS m/z (ESI) |
|---|---|---|---|---|
| **3** | 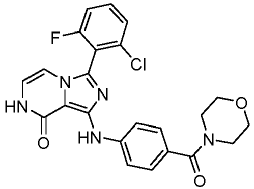 | **3f** | **1g** | 468 |
| **4** | 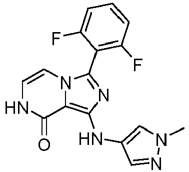 | **1f** | 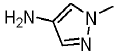 | 343 |
| **6** | 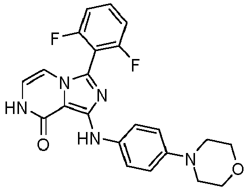 | **1f** | 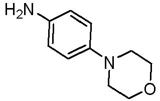 | 424 |
| 7 | 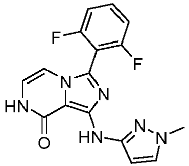 | **1f** | 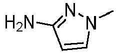 | 343 |
| **9** | 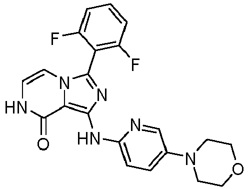 | **1f** | 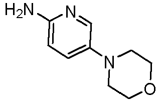 | 425 |
| **11** | 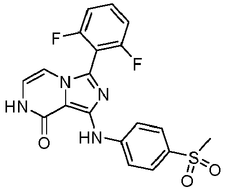 | **1f** | 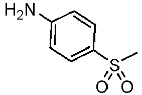 | 417 |
| **14** | 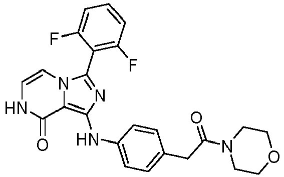 | **1f** | 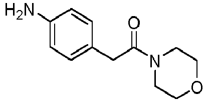 | 466 |
| **22** | 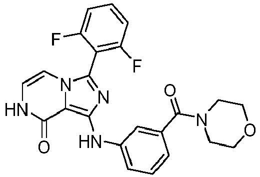 | **1f** | 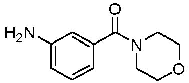 | 452 |
| **26** | 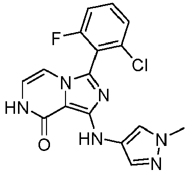 | **3f** | 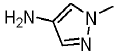 | 359 |
| **27** | 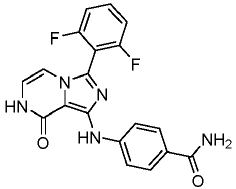 | **1f** | 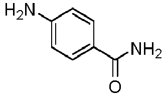 | 382 |
| **28** | 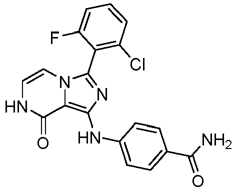 | **3f** | 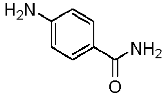 | 398 |
| **29** | 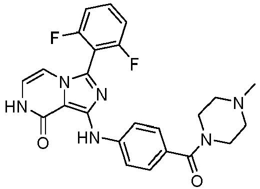 | **1f** | 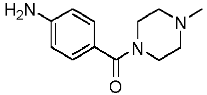 | 465 |
| **30** | 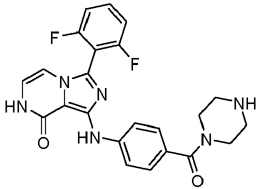 | **1f** | 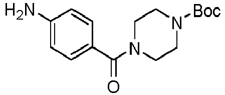 | 451 |
| **31** | 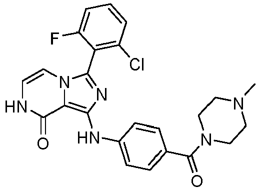 | **3f** | 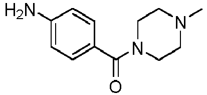 | 481 |
| **32** | 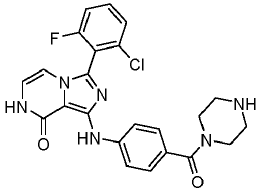 | **3f** | 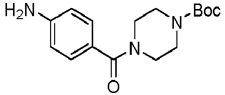 | 467 |
| **33** | 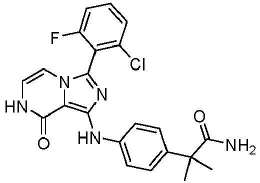 | **3f** | **33c** | 440 |
| **34** | 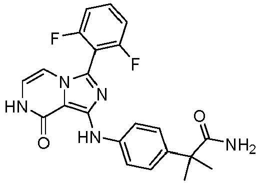 | **1f** | **33c** | 424 |
| **35** | 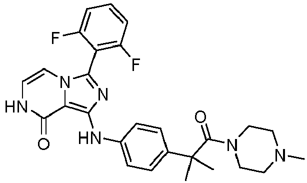 | **1f** | **35d** | 507 |
| **36** | 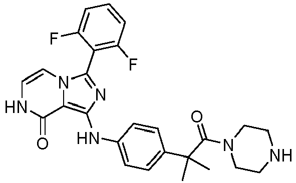 | **1f** | **36d** | 493 |
| **37** | 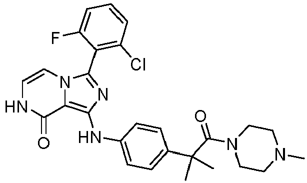 | **3f** | **35d** | 523 |
| **38** | 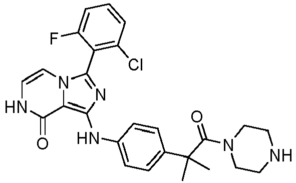 | **3f** | **36d** | 509 |
| **39a** | 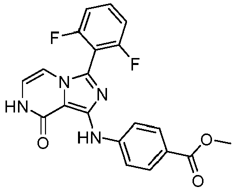 | **1f** | 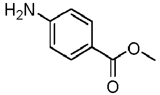 | 397 |
| **40a** | 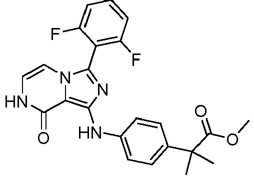 | **1f** | 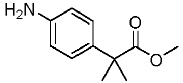 | 439 |

The nuclear magnetic resonance data of compounds **3**, **4**, **6**, **7**, **9**, **11**, **14**, **22**, **26**, **27**, **28**, **29**, **30**, **31**, **32**, **33**, **34**, **35**, **36**, **37** and **38** were as follows:

| Compound | ¹H NMR |
|---|---|
| **3-(2-chloro-6-fluorophenyl)-1-((4-(morpholine-4-carbonyl)phenyl)amino)imidazo[1,5-a]pyrazin-8(7H)-one hydrochloride (3)** | ¹H NMR(400 MHz,CD₃OD) δ 7.74 (td, *J* = 8.4, 6.1 Hz, 1H), 7.60 (dd, *J* = 12.3, 5.4 Hz, 3H), 7.49-7.39 (m, 3H), 6.84 (dd, *J* = 5.9, 1.0 Hz, 1H), 6.74 (d, *J* = 6.0 Hz, 1H), 3.72 (brs, 8H). |
| **3-(2,6-difluorophenyl)-1-((1-methyl-1H-pyrazol-4-yl)amino)imidazo[1,5-a]pyrazin-8(7H)-one hydrochloride (4)** | ¹H NMR (400 MHz, CD₃OD) δ 8.11 (s, 1H), 7.93 (s, 1H), 7.73 (tt, *J* = 8.5, 6.5 Hz, 1H), 7.28 (t, *J* = 8.3 Hz, 2H), 6.83 (d, *J* = 6.0 Hz, 1H), 6.67 (d, *J* = 6.0 Hz, 1H), 4.00 (s, 3H). |
| **3-(2,6-difluorophenyl)-1-((4-morpholinophenyl)amino)imidazo[1,5-a]pyrazin-8(7H)-one hydrochloride (6)** | ¹H NMR (400 MHz, CD₃OD) δ 7.89 (d, *J* = 8.1 Hz, 2H), 7.69 (td, *J* = 8.4, 4.2 Hz, 1H), 7.59 (d, *J* = 7.7 Hz, 2H), 7.26 (t, *J* = 8.2 Hz, 2H), 6.84 (s, 1H), 6.59 (d, *J* = 5.2 Hz, 1H), 4.10 (brs, 4H), 3.73 (brs, 4H). |
| **3-(2,6-difluorophenyl)-1-((1-methyl-1H-pyrazol-3-yl)amino)imidazo[1,5-a]pyrazin-8(7H)-one (7)** | ¹H NMR (400 MHz,DMSO-*d₆*) δ 10.40 (d, *J* = 5.2 Hz, 1H), 7.84 (s, 1H), 7.76-7.65 (m, 1H), 7.50 (d, *J* = 2.1 Hz, 1H), 7.40-7.30 (m, 2H), 6.79 (d, *J* = 5.9 Hz, 1H), 6.58-6.45 (m, 2H), 3.71 (s, 3H). |
| **3-(2,6-difluorophenyl)-1-((5-morpholinopyridin-2-yl)amino)imidazo [1,5-a]pyrazin-8(7H)-one (9)** | ¹H NMR (400 MHz,DMSO-*d₆*) δ 10.53 (d, *J* = 5.2 Hz, 1H), 8.23 (s, 1H), 8.06 (d, *J* = 9.1 Hz, 1H), 7.92 (d, *J* = 2.9 Hz, 1H), 7.79-7.66 (m, 1H), 7.45 (dd, *J* = 9.1, 2.9 Hz, 1H), 7.41-7.33 (m, 2H), 6.85 (d, *J* = 5.7 Hz, 1H), 6.63-6.55 (m, 1H), 3.81-3.65 (m, 4H), 3.11-2.98 (m, 4H). |
| **3-(2,6-difluorophenyl)-1-((4-(methylsulfonyl)phenyl)amino)imidazo[1,5-a]pyrazin-8(7H)-one hydrochloride (11)** | ¹H NMR (400 MHz,DMSO-*d₆*) δ 10.67 (brs, 1H), 8.82 (s, 1H), 7.73-7.83 (m, 5H), 7.36-7.40 (m, 3H), 6.88 (d, *J* = 5.9 Hz, 1H), 6.62 (d, *J* = 5.9 Hz, 1H), 3.11(s, 3H). |
| **3-(2,6-difluorophenyl)-1-((4-(2-morpholino-2-oxoethyl)phenyl)amino)imidazo[1,5-a]pyrazin-8(7H)-one hydrochloride (14)** | ¹H NMR (400 MHz,DMSO-*d₆*) δ 10.47 (d, *J* = 5.3 Hz, 1H), 8.02 (br, 1H), 7.79-7.67 (m, 1H), 7.58 (d, *J* = 8.5 Hz, 2H), 7.43-7.27 (m, 2H), 7.09 (d*, J* = 8.5 Hz, 2H), 6.81 (d*, J* = 5.5 Hz, 1H), 6.62-6.50 (m, 1H), 4.60-3.95 (m, 4H), 3.62 (s, 2H), 3.55-3.48 (m, 2H), 3.44-3.38 (m, 2H). |
| **3-(2,6-difluorophenyl)-1-((3-(morpholine-4-carbonyl)phenyl)amino)imidazo[1,5-a]pyrazin-8(7H)-one hydrochloride (22)** | ¹H NMR (400 MHz,DMSO-*d₆*) 610.53 (d*, J* = 5.1 Hz, 1H), 8.33 (s, 1H), 7.78 (s, 1H), 7.76-7.63 (m, 2H), 7.37 (t, *J* = 8.3 Hz, 2H), 7.31 (t, *J* = 7.8 Hz, 1H), 6.85 (dd, *J* = 14.4, 6.5 Hz, 2H), 6.58 (t, *J* = 5.6 Hz, 1H), 3.56 (s, 8H). |
| **3-(2-chloro-6-fluorophenyl)-1-((1-methyl-1H-pyrazol-4-yl)amino)imidazo[1,5-a]pyrazin-8(7H)-one hydrochloride (26)** | ¹H NMR (400 MHz,DMSO-*d₆*) δ 10.25 (d, *J* = 5.2 Hz, 1H), 7.99 (s, 1H), 7.86 (s, 1H), 7.69 (td, *J* = 8.3, 6.2 Hz, 1H), 7.57 (t, *J* = 4.0 Hz, 2H), 7.47 (t, *J* = 8.5 Hz, 1H), 6.62 (d, *J* = 5.9 Hz, 1H), 6.47 (t, *J* = 5.7 Hz, 1H), 3.76 (s, 3H). |
| **4-((3-(2,6-difluorophenyl)-8-oxo-7,8-dihydroimidazo[1,5-a]pyrazin-1-yl)amino)benzamide (27)** | ¹H NMR (400 MHz,DMSO-*d₆*) δ 10.57 (d, *J* = 5.4 Hz, 1H), 8.46 (s, 1H), 7.79-7.65 (m, 5H), 7.38 (t, *J* = 8.3 Hz, 2H), 7.08 (s, 1H), 6.85 (d, *J* = 5.9 Hz, 1H), 6.60 (d, *J* = 5.6 Hz, 1H), 1.23 (s, 1H). |
| **4-((3-(2-chloro-6-fluorophenyl)-8-oxo-7,8-dihydroimidazo[1,5-a]pyrazin-1-yl)amino)benzamide (28)** | ¹H NMR (400 MHz,DMSO-*d₆*) δ 10.56 (d, *J* = 5.2 Hz, 1H), 8.46 (s, 1H), 7.80-7.65 (m, 5H), 7.60 (d, *J* = 8.1 Hz, 1H), 7.50 (t, *J* = 8.4 Hz, 1H), 7.07 (s, 1H), 6.76 (d, *J* = 5.9 Hz, 1H), 6.57 (t, *J* = 5.7 Hz, 1H), 1.23 (s, 1H). |
| **3-(2,6-difluorophenyl)-1-((4-(4-methylpiperazine-1-carbonyl)phenyl)amino)imidazo[1,5-a]pyrazin-8(7H)-one (29)** | ¹H NMR (400 MHz,DMSO-*d₆*) δ 10.55 (d, *J* = 5.2 Hz, 1H), 8.41 (s, 1H), 7.77-7.66 (m, 3H), 7.37 (t, *J* = 8.3 Hz, 2H), 7.30 (d, *J* = 8.7 Hz, 2H), 6.84 (d, *J* = 5.7 Hz, 1H), 6.58 (t, *J* = 5.7 Hz, 1H), 3.49 (s, 4H), 2.30 (s, 4H), 2.18 (s, 3H). |
| **3-(2,6-difluorophenyl)-1-((4-(piperazine-1-carbonyl)phenyl)amino)imidazo[1,5-a]pyrazin-8(7H)-one (30)** | ¹H NMR (400 MHz,DMSO-*d₆*) δ 10.54 (s, 1H), 8.39 (s, 1H), 7.77-7.66 (m, 3H), 7.37 (t, *J* = 8.3 Hz, 2H), 7.28 (d, *J* = 8.7 Hz, 2H), 6.84 (dd, *J* = 4.3, 1.7 Hz, 1H), 6.58 (d, *J* = 5.9 Hz, 1H), 3.41 (s, 4H), 2.67 (s, 4H), 1.23 (s, 1H). |
| **3-(2-chloro-6-fluorophenyl)-1-((4-(4-methylpiperazine-1-carbonyl)phenyl)amino)imidazo[1,5-a]pyrazin-8(7H)-one (31)** | ¹H NMR (400 MHz,DMSO-*d₆*) δ 10.53 (d, *J* = 5.3 Hz, 1H), 8.41 (s, 1H), 7.68 (d, *J* = 8.7 Hz, 3H), 7.59 (d, *J* = 8.1 Hz, 1H), 7.50 (t, *J* = 8.8 Hz, 1H), 7.29 (d, *J* = 8.7 Hz, 2H), 6.75 (d, *J* = 5.9 Hz, 1H), 6.56 (t, *J* = 5.7 Hz, 1H), 3.48 (s, 4H), 2.30 (s, 4H), 2.18 (s, 3H). |
| **3-(2-chloro-6-fluorophenyl)-1-((4-(piperazine-1-carbonyl)phenyl)amino)imidazo[1,5-a]pyrazin-8(7H)-one (32)** | ¹H NMR (400 MHz,DMSO-*d₆*) δ 10.53 (s, 1H), 8.39 (s, 1H), 7.75-7.65 (m, 3H), 7.59 (d, *J* = 8.2 Hz, 1H), 7.51 (d, *J* = 9.2 Hz, 1H), 7.28 (d, *J* = 8.7 Hz, 2H), 6.75 (dd, *J* = 5.9, 1.0 Hz, 1H), 6.57 (s, 1H), 3.41 (s, 4H), 2.67 (s, 4H), 1.23 (s, 1H). |
| **2-(4-((3-(2-chloro-6-fluorophenyl)-8-oxo-7,8-dihydroimidazo[1,5-a]pyrazin-1-yl)amino)phenyl)-2-methylpropanamide (33)** | ¹H NMR (400 MHz,DMSO-*d₆*) δ 10.44 (d, *J* = 5.2 Hz, 1H), 8.06 (s, 1H), 7.70 (td, *J* = 8.3, 6.2 Hz, 1H), 7.58 (dd, *J* = 8.4, 3.7 Hz, 3H), 7.48 (dd, *J* = 13.0, 4.6 Hz, 1H), 7.21 (d, *J* = 8.8 Hz, 2H), 6.77 (d, *J* = 16.6 Hz, 2H), 6.71 (d, *J* = 5.9 Hz, 1H), 6.53 (t, *J* = 5.7 Hz, 1H), 1.40 (s, 6H). |
| **2-(4-((3-(2,6-difluorophenyl)-8-oxo-7,8-dihydroimidazo[1,5-a]pyrazin-1-yl)amino)phenyl)-2-methylpropanamide (34)** | ¹H NMR (400 MHz,DMSO-*d₆*) δ 10.46 (d, *J* = 5.3 Hz, 1H), 8.06 (s, 1H), 7.76-7.66 (m, 1H), 7.59 (d, *J* = 8.7 Hz, 2H), 7.36 (t, *J* = 8.2 Hz, 2H), 7.21 (d, *J* = 8.7 Hz, 2H), 6.80 (d, *J* = 6.6 Hz, 2H), 6.76 (s, 1H), 6.55 (t, *J* = 5.7 Hz, 1H), 1.40 (s, 6H). |
| **3-(2,6-difluorophenyl)-1-((4-(2-methyl-1-(4-methylpiperazin-1-yl)-1-oxopropan-2-yl)phenyl)amino)imidazo[1,5-a]pyrazin-8(7H)-one (35)** | ¹H NMR (400 MHz,DMSO-*d₆*) δ 10.47 (d, *J* = 5.4 Hz, 1H), 8.12 (s, 1H), 7.72 (dd, *J* = 11.8, 5.2 Hz, 1H), 7.64 (d, *J* = 8.7 Hz, 2H), 7.37 (t, *J* = 8.3 Hz, 2H), 7.06 (d, *J* = 8.7 Hz, 2H), 6.81 (d, *J* = 5.5 Hz, 1H), 6.56 (t, *J* = 5.7 Hz, 1H), 3.27-2.84 (m, 4H), 2.04 (s, 3H), 2.03-1.68 (m, 4H), 1.39 (s, 6H). |
| **3-(2,6-difluorophenyl)-1-((4-(2-methyl-1-oxo-1-(piperazin-1-yl)propan-2-yl)phenyl)amino)imidazo[1,5-a]pyrazin-8(7H)-one (36)** | ¹H NMR (400 MHz,DMSO-*d₆*) δ 10.47 (s, 1H), 8.11 (s, 1H), 7.77-7.68 (m, 1H), 7.64 (d, *J* = 8.7 Hz, 2H), 7.37 (t, *J* = 8.3 Hz, 2H), 7.06 (d, *J* = 8.7 Hz, 2H), 6.81 (dd, *J* = 4.2, 1.7 Hz, 1H), 6.56 (d, *J* = 5.9 Hz, 1H), 3.28-2.80 (m, 4H), 2.46-2.08 (m, 4H), 1.38 (s, 6H). |
| **3-(2-chloro-6-fluorophenyl)-1-((4-(2-methyl-1-(4-methylpiperazin-1-yl)-1-oxopropan-2-yl)phenyl)amino)imidazo[1,5-a]pyrazin-8(7H)-one (37)** | ¹H NMR (400 MHz,DMSO-*d₆*) δ 10.46 (d, *J* = 5.4 Hz, 1H), 8.12 (s, 1H), 7.71 (d, *J* = 6.1 Hz, 1H), 7.63 (d, *J* = 8.7 Hz, 2H), 7.59 (d, *J* = 8.1 Hz, 1H), 7.49 (s, 1H), 7.06 (d, *J* = 8.7 Hz, 2H), 6.72 (d, *J* = 5.9 Hz, 1H), 6.54 (t, *J* = 5.7 Hz, 1H), 3.25-2.95 (m, 4H), 2.05 (s, 3H), 2.03 - 1.74 (m, 4H), 1.38 (s, 6H). |
| **3-(2-chloro-6-fluorophenyl)-1-((4-(2-methyl-1-oxo-1-(piperazin-1-yl)propan-2-yl)phenyl)amino)imidazo[1,5-a]pyrazin-8(7H)-one (38)** | ¹H NMR (400 MHz,DMSO-*d₆*) δ 10.46 (s, 1H), 8.11 (s, 1H), 7.70 (dd, *J* = 8.3, 6.2 Hz, 1H), 7.63 (d, *J* = 8.7 Hz, 2H), 7.59 (d, *J* = 8.2 Hz, 1H), 7.49 (t, *J* = 8.8 Hz, 1H), 7.06 (d, *J* = 8.7 Hz, 2H), 6.72 (d, *J* = 4.9 Hz, 1H), 6.54 (s, 1H), 3.23-2.95 (m, 4H), 2.33 (s, 4H), 1.38 (s, 6H). |

### Example 5

### 3-(2,6-difluorophenyl)-1-((4-(morpholine-4-carbonyl)phenyl)amino)-6,7-dihydroimidazo [1,5-a]pyrazine-8(5H)-one (Compound 2)

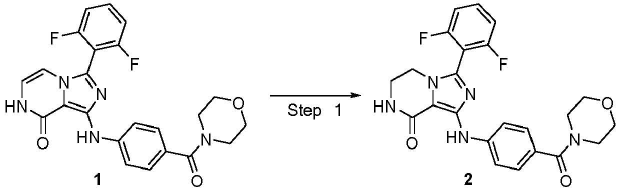

**1** (97mg, 0.215mmol), methanol (30mL), and 10% palladium/carbon (97 mg) were mixed. The resulting mixture was heated to 30°C under an atmosphere of hydrogen gas, stirred for 16 hours, and filtered. The filtrate was concentrated to dryness. The residue was purified with a reversed-phase preparative high-performance liquid chromatography to produce the target product **2** (solid, 25mg, 26%).
MS m/z (ESI): 490 [M+1]
¹H NMR (400 MHz, CD₃OD) δ 7.73 (d, *J* = 6.3 Hz, 1H), 7.66 (td, *J* = 6.2, 2.8 Hz, 3H), 7.58 (d, *J* = 8.3 Hz, 2H), 7.28 (ddd, *J* = 9.1, 7.6, 3.3 Hz, 1H), 7.19 (dd, *J* = 9.1, 4.2 Hz, 1H), 6.94 (d, *J* = 6.4 Hz, 1H), 4.73 (s, 2H), 4.52 (t, *J* = 11.5 Hz, 1H), 3.99 (s, 3H), 3.76-3.70 (m, 1H), 2.21-2.03 (m, 6H), 1.63-1.53 (m, 2H).

The compounds in the following table were all prepared according to the experimental steps of Example 5 except that different compounds were respectively used to replace 1 in the preparation.

| Compound No. | Compound structure | Compound replacing 1 | MS m/z (ESI) |
|---|---|---|---|
| **5** | 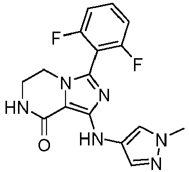 | **4** | 345 |
| **8** | 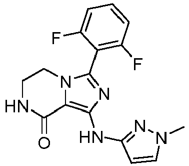 | **7** | 345 |
| **10** | 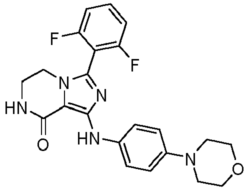 | **6** | 426 |
| **12** | 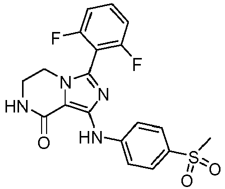 | **11** | 419 |
| **16** | 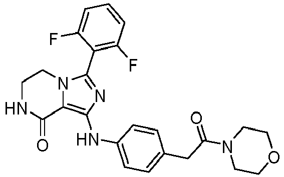 | **14** | 468 |
| **18** | 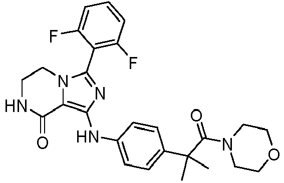 | **17** | 496 |
| **23** | 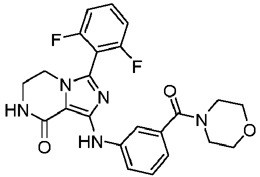 | **22** | 454 |

The nuclear magnetic resonance data of compounds **5**, **8**, **10**, **12**, **16**, **18**, and **23** were as follows:

| Compound | ¹H NMR |
|---|---|
| **3-(2,6-difluorophenyl)-1-((1-methyl-1H-pyrazol-4-yl)amino)-6,7-dihydroimidazo[1,5-a]pyrazin-8(5H)-one hydrochloride (5)** | ¹H NMR(400 MHz, CD₃OD) δ7.99 (s, 1H), 7.86-7.78 (m, 2H), 7.33 (t, *J* = 8.5 Hz, 2H), 4.30-4.24 (m, 2H), 3.99 (s, 3H), 3.76-3.71 (m, 2H). |
| **3-(2,6-difluorophenyl)-1-((1-methyl-1H-pyrazol-3-yl)amino)-6,7-dihydroimidazo[1,5-a]pyrazin-8(5H)-one (8)** | ¹H NMR (400 MHz, DMSO-*d₆*) δ 7.85 (s, 1H), 7.74 (s, 1H), 7.72-7.63 (m, 1H), 7.46 (d, *J* = 2.1 Hz, 1H), 7.36-7.28 (m, 2H), 6.45 (d, *J* = 2.2 Hz, 1H), 4.00-3.93 (m, 2H), 3.69 (s, 3H), 3.52-3.44 (m, 2H). |
| **3-(2,6-difluorophenyl)-1-((4-morpholinophenyl)amino)-6,7-dihydroimidazo[1,5-a]pyrazin-8(5H)-one (10)** | ¹H NMR (400 MHz, DMSO-*d₆*) δ 7.80 (s, 1H), 7.72 (s, 1H), 7.71-7.66 (m, 1H), 7.49 (d, *J* = 9.0 Hz, 2H), 7.33 (t, *J* = 8.2 Hz, 2H), 6.86 (d, *J* = 9.1 Hz, 2H), 3.99-3.93 (m, 2H), 3.75-3.68 (m, 4H), 3.48 (s, 2H), 3.01-2.95 (m, 4H). |
| **3-(2,6-difluorophenyl)-1-((4-(methylsulfonyl)phenyl)amino)-6,7-dihydroimidazo[1,5-a]pyrazin-8(5H)-one hydrochloride (12)** | ¹H NMR (400 MHz, DMSO-*d₆*) δ 8.05 (s, 1H), 7.95 (s, 1H), 7.71-7.79 (m, 5H), 7.34-7.38 (m, 2H), 4.02 (s, 2H), 3.52 (s, 2H), 3.11(s, 3H). |
| **3-(2,6-difluorophenyl)-1-((4-(2-morpholino-2-oxoethyl)phenyl)amino)-6,7-dihydroimidazo[1,5-a]pyrazin-8(5H)-one hydrochloride (16)** | ¹H NMR (400 MHz, DMSO-*d₆*) δ 8.30-7.85 (brs, 1H), 7.81 (s, 1H), 7.74-7.64 (m, 1H), 7.53 (d, *J* = 8.6 Hz, 2H), 7.39-7.29 (m, 2H), 7.08 (d, *J* = 8.6 Hz, 2H), 4.31-3.74 (m, *J* = 42.2, 36.1 Hz, 6H), 3.61 (s, 2H), 3.56-3.48 (m, 4H), 3.43-3.38 (m, 2H). |
| **3-(2,6-difluorophenyl)-1-((4-(2-methyl-1-morpholino-1-oxopropan-2-yl)phenyl)amino)-6,7-dihydroimidazo [1,5-a]pyrazin-8(5H)-one hydrochloride (18)** | ¹H NMR (400 MHz, DMSO-*d₆*) δ 8.04 (s, 1H), 7.81 (s, 1H), 7.75-7.64 (m, 1H), 7.60 (d, *J* = 8.7 Hz, 2H), 7.41-7.29 (m, 2H), 7.06 (d, *J* = 8.7 Hz, 2H), 3.98 (t, *J* =4.8 Hz, 2H), 3.59-3.35 (m, 10H), 1.39 (s, 6H). |
| **3-(2,6-difluorophenyl)-1-((3-(morpholine-4-carbonyl)phenyl)amino)-6,7-dihydroimidazo[1,5-a]pyrazin-8(5H)-one hydrochloride (23)** | ¹H NMR (400 MHz, DMSO-*d₆*) δ 8.21 (s, 1H), 7.85 (s, 1H), 7.76 (s, 1H), 7.66-7.70 (m, 1H), 7.60 (dd, *J* = 8.2, 1.4 Hz, 1H), 7.27-7.37 (m, 3H), 6.84 (d, *J* = 7.5 Hz, 1H), 4.09-3.92 (m, 2H), 3.51-3.55(m, 6H), 3.48-3.51(m, 4H). |

### Example 6

### 1-((4-(1-(azetidin-1-yl)-2-methyl-1-oxopropan-2-yl)phenyl)amino)-3-(2,6-difluorophenyl)-6,7-dihydroimidazo[1,5-a]pyrazin-8(5H)-one (Compound 13)

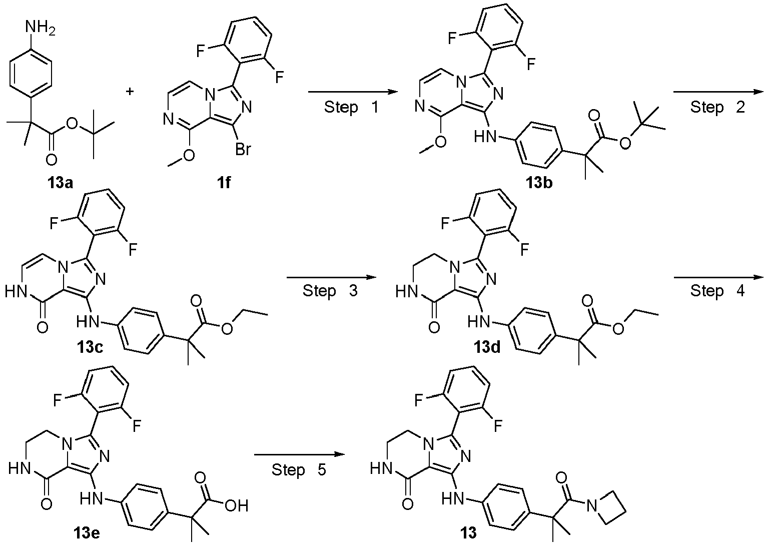

### Step 1

### Tert-butyl 2-(4-((3-(2,6-difluorophenyl)-8-methoxyimidazo[1,5-a]pyrazin-1-yl)amino)phenyl)-2-methylpropanoate (13b)

To a solution of **1f** (200mg, 0.588mmol) in 1,4-dioxane (5mL) were added *tert*-butyl 2-(4-aminophenyl)-2-methylpropanoate **13a** (280mg, 1. 17mmol), Brettphos (62mg, 0.118mmol), Pd₂(dba)₃ (53mg, 0.058mmol) and cesium carbonate (600mg, 1.76mmol) under a nitrogen gas atmosphere. Then the resulting mixture was heated in a microwave reactor to 90°C and stirred for 1 hour. After cooling to room temperature, the mixture was concentrated to dryness under a reduced pressure. The residue was purified with a silica gel column chromatography (petroleum ether/ethyl acetate = 100/0 to 7/3) to produce the target product **13b** (200mg, 68%).

MS m/z (ESI): 495 [M+1]

### Step 2

### Ethyl 2-(4-((3-(2,6-difluorophenyl)-8-oxo-7,8-dihydroimidazo[1,5-a]pyrazin-1-yl)amino)phenyl)-2-methylpropanoate (13c)

To a solution of **13b** (100mg, 0.1mmol) in ethanol (4mL) was added a solution of HCl in ethanol (12N, 2mL). The solution was stirred at 70°C for 2 hours, then cooled to room temperature, and concentrated to dryness to produce the target product **13c** (90 mg). The product was not further purified and directly used in the next step reaction.

MS m/z (ESI): 453 [M+1]

### Step 3

### Ethyl 2-(4-((3-(2,6-difluorophenyl)-8-oxo-5,6,7,8-tetrahydroimidazo[1,5-a]pyrazin-1-yl)amino)phenyl)-2-methylpropanoate (13d)

13c (90mg, 0.2mmol), ethanol (5mL), and 10% palladium/carbon (100 mg) were mixed. The resulting mixture was stirred for 3 hours under an atmosphere of hydrogen gas, and filtered. The filtrate was concentrated to dryness to produce the target product **13d** (80mg, 88%).

MS m/z (ESI): 455 [M+1]

### Step 4

### 2-(4-((3-(2,6-difluorophenyl)-8-oxo-5,6,7,8-tetrahydroimidazo[1,5-a]pyrazin-1-yl)amino)phenyl)-2-methylpropanoic acid (13e)

To a solution of **13d** (90mg, 0.2mmol) in tetrahydrofuran (5mL) was added lithium hydroxide monohydrate (17mg, 0.4mmol). The resulting mixture was heated to 60°C and stirred for 18 hours. After cooling to room temperature, the reaction solution was concentrated to dryness. The residue was purified with a reversed-phase preparative high-performance liquid chromatography to produce the target product **13e** (60mg, containing TFA).

MS m/z (ESI): 427 [M+1]

### Step 5

### 1-((4-(1-(azetidin-1-yl)-2-methyl-1-oxopropan-2-yl)phenyl)amino)-3-(2,6-difluorophenyl)-6,7-dihydroimidazo[1,5-a]pyrazin-8(5H)-one (13)

To a mixture of **13e** (17mg, 0.04mmol), DMF (2mL), HATU (20mg, 0.052mmol), and azetidine (10mg, 0.12mmol) was added diisopropylethylamine (26mg, 0.2mmol). The resulting solution was stirred at room temperature for 1 hour, and then directly purified with a reversed-phase preparative high-performance liquid chromatography to produce the target product **13** (solid, 2.69mg, 14%).
MS m/z (ESI): 466 [M+1]
¹H NMR (400 MHz, DMSO-*d₆*) δ 8.04 (s, 1H), 7.81 (s, 1H), 7.75-7.65 (m, 1H), 7.59 (d, *J* = 8.7 Hz, 2H), 7.42-7.29 (m, 2H), 7.10 (d, *J* = 8.7 Hz, 2H), 4.06-3.93 (m, 2H), 3.86-3.71 (m, 2H), 3.53-3.47 (m, 2H), 2.59-2.51 (m, 2H), 2.03-1.84 (m, 2H), 1.37 (s, 6H).

### Example 7

### 3-(2-chloro-6-fluorophenyl)-1-((4-(morpholine-4-carbonyl)phenyl)amino)-6,7-dihydroimidazo[1,5-a]pyrazin-8(5H)-one (Compound 15)

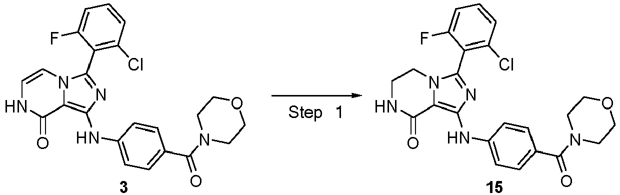

To a solution of **3** (140mg, 0.3mmol) in ethanol (10mL) were added chlorobenzene (1mL) and platinum dioxide (70 mg). The mixture was stirred under an atmosphere of hydrogen gas for 70 minutes, and then filtered. The filtrate was concentrated to dryness. The residue was purified with a reversed-phase preparative high-performance liquid chromatography to produce the target product **15** (solid, 62.7mg, 44%).
MS m/z (ESI): 312 [M+1]
¹H NMR (400 MHz, DMSO-*d₆*) δ 8.29 (s, 1H), 7.87 (s, 1H), 7.72-7.63 (m, 3H), 7.57 (d, *J* = 8.1 Hz, 1H), 7.47 (t, *J* = 8.7 Hz, 1H), 7.31 (d, *J* = 8.6 Hz, 2H), 3.95-3.90 (m, 2H), 3.61-3.55 (m, 4H), 3.54-3.45 (m, 6H).

### Example 8

### 3-(2,6-difluorophenyl)-1-((4-(2-methyl-1-morpholino-1-oxopropan-2-yl)phenyl)amino)imidazo[1,5-a]pyrazin-8(7H)-one (compound 17)

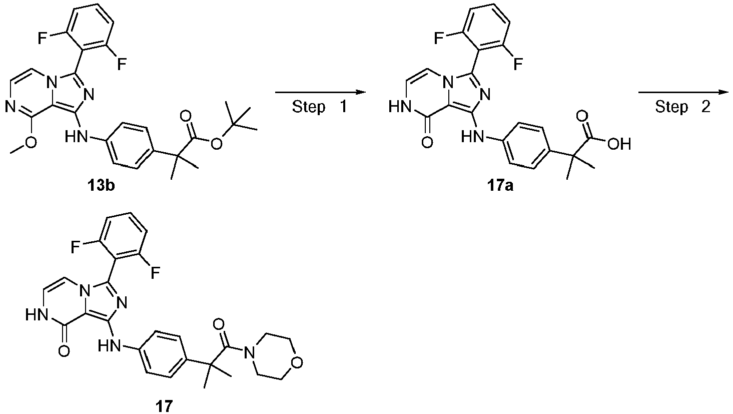

### Step 1

### 2-(4-((3-(2,6-difluorophenyl)-8-oxo-7,8-dihydroimidazo[1,5-a]pyrazin-1-yl)amino)phenyl)-2-methylpropanoic acid (17a)

A solution of **13b** (420mg, 0.848mmol) in trifluoroacetic acid (10mL) was heated to 70°C and stirred for 1 hour. After cooling to room temperature, the reaction solution was concentrated to dryness under a reduced pressure to produce the target product **17a** (360 mg). The product was not further purified and directly used in the next step reaction.

MS m/z (ESI): 425 [M+1]

### Step 2

### 3-(2,6-difluorophenyl)-1-((4-(2-methyl-1-morpholino-1-oxopropan-2-yl)phenyl)amino)imidazo[1,5-a]pyrazin-8(7H)-one hydrochloride (17)

**17a** (360mg, 0.85mmol), DMF (5mL), HATU (420mg, 1.1mmol), morpholine (220mg, 2.55mmol) and DIPEA (442mg, 3.2mmol) were mixed. The reaction mixture was stirred at room temperature for 1 hour, and directly purified with a reversed-phase preparative high-performance liquid chromatography to produce the target product **17** (solid, 320mg, 76%, hydrochloride).
MS m/z (ESI): 494 [M+1]
¹H NMR (400 MHz, DMSO-*d₆*) δ 10.47 (d, *J* = 5.3 Hz, 1H), 8.12 (s, 1H), 7.78-7.67 (m, 1H), 7.64 (d, *J* = 8.7 Hz, 2H), 7.42-7.31 (m, 2H), 7.08 (d, *J* = 8.7 Hz, 2H), 6.81 (d, *J* = 5.5 Hz, 1H), 6.62-6.49 (m, 1H), 3.69-3.41 (m, 8H), 1.40 (s, 6H).

### Example 9

### 3-(1-acetylpiperidin-4-yl)-1-((4-(morpholine-4-carbonyl)phenyl)amino)imidazo[1,5-a]pyrazin-8(7H)-one (compound 19)

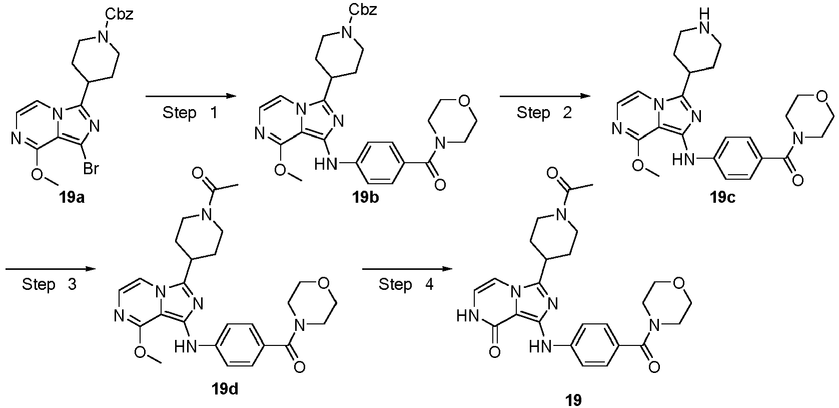

### Step 1

### Benzyl 4-(8-methoxy-1-((4-(morpholine-4-carbonyl)phenyl)amino)imidazo[1,5-a]pyrazin-3-yl)piperidine-1-carboxylate (19b)

To a mixture of **19a** (206mg, 1mmol), 1,4-dioxane (8mL), and cesium carbonate (813mg, 2.5mmol) were added XantPhos (116mg, 0.2mmol) and Pd₂(dba)₃ (92mg, 0.1mmol). The mixture was heated to 90°C in a microwave reactor and stirred for 1 hour, cooled to room temperature, and filtered. The filtrate was concentrated to dryness. The residue was purified with a silica gel column chromatography (dichloromethane/methanol=100/0 to 19/1) to produce the target product **19b** (490mg, 86%).

MS m/z (ESI): 571 [M+1]

### Step 2

### (4-((8-methoxy-3-(piperidin-4-yl)imidazo[1,5-a]pyrazin-1-yl)amino)phenyl)(morpholino)methanone (19c)

To a solution of **19b** (230mg, 0.403mmol) in methanol (10mL) was added 10% palladium/carbon (115 mg). The mixture was stirred for 30 minutes under an atmosphere of hydrogen gas, and then filtered. The filtrate was concentrated to dryness to produce the target product **19c** (190 mg). The product was not further purified and directly used in the next step reaction.

MS m/z (ESI): 437 [M+1]

### Step 3

### 1-(4-(8-methoxy-1-((4-(morpholine-4-carbonyl)phenyl)amino)imidazo[1,5-a]pyrazin-3-yl)piperidine-1-yl)ethan-1-one (19d)

To a solution of **19c** (122mg, 0.279mmol) in methylene chloride (5mL) was added triethylamine (85mg, 0837mmol). The resulting mixture was cooled to 0°C, and acetyl chloride (22mg, 0.279mmol) was added dropwise. The mixture was stirred at room temperature for 30 minutes, and concentrated to dryness. The residue was purified with a silica gel column chromatography (dichloromethane/methanol= 100/0 to 19/1) to produce the target product **19d** (81mg, 61%).

MS m/z (ESI): 479 [M+1]

### Step 4

### 3-(1-acetylpiperidin-4-yl)-1-((4-(morpholine-4-carbonyl)phenyl)amino)imidazo[1,5-a]pyrazin-8(7H)-one (19)

To a solution of **19d** (81mg, 0.17mmol) in acetonitrile (2mL) was added an HCl/ethanol solution (33%, 1mL). Then the resulting mixture was heated to 50°C and stirred for 30 minutes. The reaction mixture was cooled to room temperature, and concentrated to dryness to produce a yellow solid (85 mg). 45mg of the obtained solid was taken out, and purified with a reversed-phase preparative high-performance liquid chromatography to produce the target product **19** (solid, 31.1mg, 69%).
MS m/z (ESI): 465 [M+1]
¹H NMR (400 MHz, CD₃OD) δ 7.50-7.42 (m, 3H), 7.34-7.28 (m, 2H), 6.88 (d, *J* = 6.1 Hz, 1H), 4.72 (d, *J* = 13.5 Hz, 1H), 4.14 (d, *J* = 13.8 Hz, 1H), 3.85-3.57 (m, 9H), 3.38 (dd, *J* = 19.4, 7.5 Hz, 1H), 2.88 (t, *J* = 11.9 Hz, 1H), 2.19 (s, 3H), 2.17-2.06 (m, 2H), 1.97-1.75 (m, 2H).

### Example 10

### 1-((4-(morpholine-4-carbonyl)phenyl)amino)-3-(piperidin-4-yl)imidazo[1,5-a]pyrazin-8(7H)-one (compound 20)

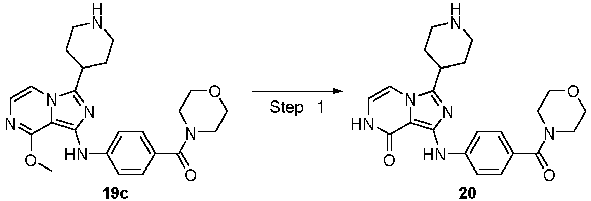

To a solution of **19c** (40mg, 0.092mmol) in acetonitrile (2mL) was added an HCl solution (30% of the solution in ethanol, 1mL). The resulting mixture was heated to 50°C and stirred for 30 minutes, then cooled to room temperature and concentrated to dryness. The residue was purified with a reversed-phase preparative high-performance liquid chromatography to produce the target product **20** (solid, 10.3mg, 25%).
MS m/z (ESI): 423 [M+1]
¹H NMR (400 MHz, CD₃OD) δ 7.66-7.61 (m, 2H), 7.44-7.39 (m, 2H), 7.28 (d, *J* = 6.1 Hz, 1H), 6.63 (d, *J* = 6.0 Hz, 1H), 3.72 (s, 8H), 3.62 (t, *J* = 3.5 Hz, 1H), 3.58 (t, *J* = 3.5 Hz, 1H), 3.56-3.49 (m, 1H), 3.30-3.20 (m, 2H), 2.29-2.17 (m, 4H).

### Example 11

### 3-(1-cyclopropylpiperidin-4-yl)-1-((4-(morpholine-4-carbonyl)phenyl)amino)imidazo [1,5-a]pyrazin-8(7H)-one (compound 21)

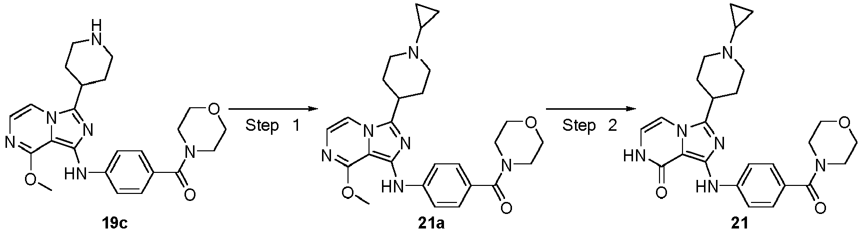

### Step 1

### (4-((3-(1-cyclopropylpiperidin-4-yl)-8-methoxyimidazo[1,5-a]pyrazin-1-yl)amino)phenyl)(morpholino)methanone (21a)

To a mixture of **19c** (150mg, 0.344mmol), methanol (2mL) and tetrahydrofuran (2mL) was added (1-ethoxycyclopropoxy)trimethylsilane (120mg, 0.687mmol), and then added acetic acid (103mg, 1.72mmol) and sodium cyanoborohydride (32mg, 0.86mmol). The mixture was heated to 60°C, stirred for 20 hours, and then cooled to room temperature. A saturated sodium bicarbonate solution (20mL) was added. The resulting mixture was extracted with ethyl acetate (3×20mL). The combined organic phases were washed with saturated salt water (20mL), dried over anhydrous sodium sulfate, and filtered. The filtrate was concentrated to dryness. The residue was purified with a silica gel column chromatography (dichloromethane/methanol= 100/0 to 19/1) to produce the target product **21a** (46mg, 28%).

MS m/z (ESI): 477 [M+1]

### Step 2

### 3-(1-cyclopropylpiperidin-4-yl)-1-((4-(morpholine-4-carbonyl)phenyl)amino)imidazo[1,5-a]pyrazin-8(7H)-one (21)

To a solution of **21a** (46mg, 0.0965mmol) in acetonitrile (2mL) was added a hydrogen chloride solution (30% of the solution in ethanol, 1mL). The resulting mixture was heated to 50°C and stirred for 30 minutes, cooled to room temperature, and concentrated to dryness. The residue was purified with a reversed-phase preparative high-performance liquid chromatography to produce the target product **21** (solid, 16.9mg, 38%).
MS m/z (ESI): 463 [M+1]
¹H NMR (400 MHz, CD₃OD) δ 7.77 (d, *J* = 8.6 Hz, 2H), 7.38 (d, *J* = 8.6 Hz, 2H), 7.15 (d, *J* = 6.0 Hz, 1H), 6.46 (d, *J* = 6.0 Hz, 1H), 3.71 (s, 4H), 3.68 (s, 4H), 3.28 (d, *J* = 11.9 Hz, 2H), 3.15-3.06 (m, 1H), 2.57 (t, *J* = 10.5 Hz, 2H), 2.13-1.94(m, 4H), 1.90 (s, 1H), 0.66-0.49 (m, 4H).

### Example 12

### 3-(2,6-difluorophenyl)-1-((1-(2-morpholino-2-oxoethyl)-1H-pyrazol-4-yl)amino)-6,7-dihydroimidazo[1,5-a]pyrazin-8(5H)-one (compound 24)

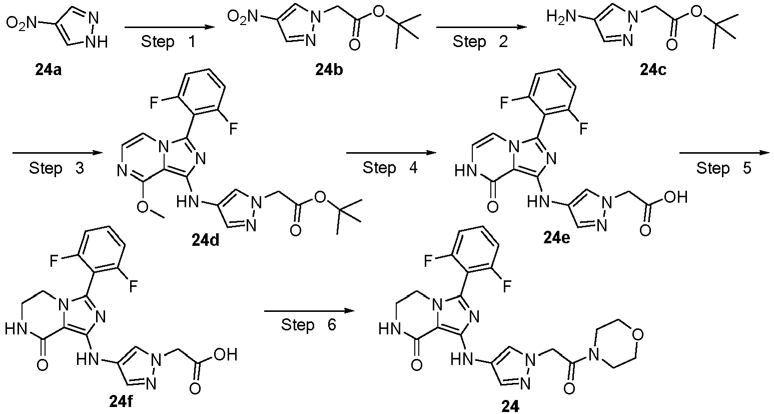

### Step 1

### Tert-butyl 2-(4-nitro-1H-pyrazol-1-yl)acetate (24b)

To a mixture of 4-nitro-1H-pyrazole 24a (2g, 17.7mmol), potassium carbonate (4.8g, 35mmol) and DMF (10mmol) was added tert-butyl 2-bromoacetate (4g, 17.7mmol) at room temperature. The resulting mixture was stirred at room temperature for 12 hours, diluted with water (200mL), and then extracted with ethyl acetate (2×150mL). The combined organic phases were washed with saturated salt water (2×150mL), then dried over anhydrous sodium sulfate, and filtered. The filtrate was concentrated to dryness under a reduced pressure. The residue was purified with a silica gel column chromatography (dichloromethane/methanol=100/0 to 99/1) to produce the target product **24b** (5g, 110%).

MS m/z (ESI): 228 [M+1]

### Step 2

### Tert-butyl 2-(4-amino-1H-pyrazol-1-yl)acetate (24c)

To a solution of **24b** (5g, 20mmol) in ethanol (20mL) was added 10% palladium/carbon (700 mg). The resulting mixture was stirred for 12 hours under an atmosphere of hydrogen gas, and then filtered. The filtrate was concentrated to dryness under a reduced pressure to produce the target product **24c** (3.45g, 77%).

MS m/z (ESI): 198 [M+1]

### Step 3

### Tert-butyl 2-(4-((3-(2,6-difluorophenyl)-8-methoxyimidazo[1,5-a]pyrazin-1-yl)amino)-1H-pyrazol-1-yl)acetate (24d)

A mixture of **1f** (600mg, 1.77mmol), 1,4-dioxane (15mL), **24c** (720mg, 3.53mmol), Brettphos (192mg, 0.353mmol), Pd₂(dba)₃ (180mg, 0.177mmol) and cesium carbonate (1.7g, 5.32mmol) was heated to 90°C under a nitrogen gas atmosphere in a microwave reactor and stirred for 1 hour. After cooling to room temperature, the solvent was removed under reduced pressure, and the residue was purified with a silica gel column chromatography (petroleum ether/ethyl acetate=100/0 to 1/4) to produce the target product **24d** (450mg, 47%).

MS m/z (ESI): 457 [M+1]

### Step 4

### 2-(4-((3-(2,6-difluorophenyl)-8-oxo-7,8-dihydroimidazo[1,5-a]pyrazin-1-yl)amino)-1H-pyrazol-1-yl)acetic acid (24e)

A solution of **24d** (450mg, 0.98mmol) in trifluoroacetic acid (10mL) was heated to 80°C and stirred for 12 hours. After cooling to room temperature, the reaction solution was concentrated to dryness under a reduced pressure to produce the target product **24e** (815 mg). The product was not further purified and directly used in the next step reaction.

MS m/z (ESI): 387 [M+1]

### Step 5

### 2-(4-((3-(2,6-difluorophenyl)-8-oxo-5,6,7,8-tetrahydroimidazo[1,5-a]pyrazin-1-yl)amino)-1H-pyrazol-1-yl)acetic acid (24f)

To a solution of **24e** (800mg, 2.07mmol) in ethanol (30mL) was added 10% palladium/carbon (300 mg). The resulting mixture was stirred for 12 hours under an atmosphere of hydrogen gas, and filtered. The filtrate was concentrated to dryness under a reduced pressure to produce the target product **24f** (280mg, 35%).

MS m/z (ESI): 389 [M+1]

### Step 6

### 3-(2,6-difluorophenyl)-1-((1-(2-morpholino-2-oxoethyl)-1H-pyrazol-4-yl)amino)-6,7-dihydroimidazo[1,5-a]pyrazin-8(5H)-one (24)

To **24f**(10mg, 0.0257mmol), HATU (13mg, 0.0335mmol), morpholine (0.025mL), and DMF (0.5mL) was added DIPEA (10mg, 0.0771mmol). The reaction mixture was stirred at room temperature for 1 hour, and directly purified with a reversed-phase preparative high-performance liquid chromatography to produce the target product **24** (solid, 2.12mg, 18%).
MS m/z (ESI): 458 [M+1]
¹H NMR (400 MHz, DMSO-*d₆*) δ 7.84-7.81 (m, 1H), 7.80 (s, 1H), 7.72-7.62 (m, 2H), 7.55 (s, 1H), 7.36-7.29 (m, 2H), 5.03 (s, 2H), 3.97-3.90 (m, 2H), 3.60-3.52 (m, 2H), 3.50-3.39 (m, 8H).

Compound **25** was prepared according to the experimental steps of Example 12 except that in step 6, N-methylpiperazine was used to replace morpholine.

| Compound No. | Compound structure | Compound replacing morpholine | MS m/z (ESI) |
|---|---|---|---|
| **25** | 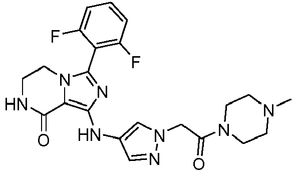 | N-methylpiperazine | 471 |

The nuclear magnetic resonance data of compound25was as follows:

| Compound | ¹H NMR |
|---|---|
| **3-(2,6-difluorophenyl)-1-((1-(2-(4-methylpiperazin-1-yl)-2-oxoethyl)-1H-pyrazol-4-yl)amino)-6,7-dihydroimidazo[1,5-a]pyrazin-8(5H)-one (25)** | ¹H NMR (400 MHz, DMSO-*d₆*) δ 7.81 (s, 1H), 7.79 (s, 1H), 7.72-7.62 (m, 2H), 7.54 (s, 1H), 7.36-7.29 (m, 2H), 5.01 (s, 2H), 3.97-3.89 (m, 2H), 3.50-3.45 (m, 2H), 3.45-3.39 (m, 4H), 2.31-2.21 (m, 4H), 2.16 (s, 3H). |

### Example 13

### 4-((3-(2,6-difluorophenyl)-8-oxo-7,8-dihydroimidazo[1,5-a]pyrazin-1-yl)amino)benzoic acid (Compound 39)

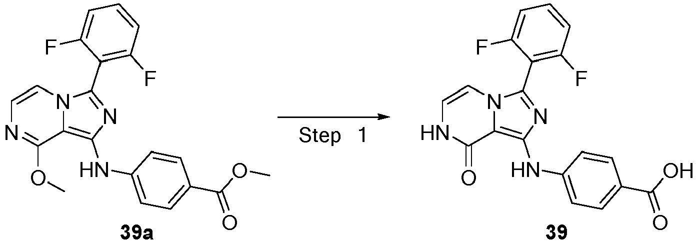

To a mixed solution of **39a** (150mg, 0.37mmol) in methanol, tetrahydrofuran, and water (3/3/1 v/v/v, 7mL) was added lithium hydroxide monohydrate (155mg, 3.7mmol). The resulting mixture was heated to 50°C and stirred for 12 hours. After cooling to room temperature, the mixture was concentrated under a reduced pressure to remove an organic solvent. The residue was adjusted to pH=1 with 1N hydrochloric acid, stirred at room temperature for 5 hours, and then concentrated to dryness. The residue was purified with a reversed-phase preparative high-performance liquid chromatography to produce the target product **39** (solid, 25mg, 18%).
MS m/z (ESI): 383 [M+1]
¹H NMR (400 MHz, DMSO-*d₆*) δ 12.36 (s, 1H), 10.61 (d, *J* = 5.5 Hz, 1H), 8.62 (s, 1H), 7.81 (d, *J* = 8.8 Hz, 2H), 7.70 (d, *J* = 8.9 Hz, 3H), 7.39 (d, *J* = 8.2 Hz, 2H),6.87 (d, *J* = 5.6 Hz, 1H), 6.60 (s, 1H).

Compound **40** was prepared according to the experimental steps of Example 13 except that **40a** was used to replace **39a** in the preparation.

| Compound No. | Compound structure | Compound replacing39a | MS m/z (ESI) |
|---|---|---|---|
| **40** | 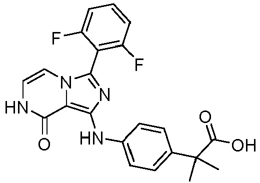 | **40a** | 425 |

The nuclear magnetic resonance data of compound **40** was as follows:

| Compound | ¹H NMR |
|---|---|
| **2-(4-((3-(2,6-difluorophenyl)-8-oxo-7,8-dihydroimidazo[1,5-a]pyrazin-1-yl)amino)phenyl)-2-methylpropanoic acid(40)** | ¹H NMR (400 MHz, DMSO-*d₆*) δ 12.18 (s, 1H), 10.47 (d, *J* = 5.3 Hz, 1H), 8.09 (s, 1H), 7.77-7.67 (m, 1H), 7.60 (d, *J* = 8.8 Hz, 2H), 7.36 (dd, *J* = 13.7, 5.4 Hz, 2H), 7.22 (d, *J* = 8.8 Hz, 2H), 6.81 (d, *J* = 5.2 Hz, 1H), 6.55 (t, *J* = 5.7 Hz, 1H), 1.44 (s, 6H). |

### Biological assays

### Example 14

### JAK2 Activity Inhibition Assay

The effect of the compounds of the present invention on the activity of JAK2 was assessed by using an in vitro kinase detection assay.

The assay method was generally described below:
The enzymatic activity of JAK2 was assessed by detecting the substrate phosphorylation level in a kinase reaction with a homogeneous time-resolved fluorescence (HTRF) kinase detection kit (Cisbio, 62TK0PEC). A reaction buffer contained an enzyme buffer (1×), 5 mM MgCl₂, 1 mM DTT and 0.01%Brij35 from the kit; a human recombinant JAK2 protein (Carna Biosciences, 08-045) was diluted to a kinase reaction solution of 0.15 ng/µL with the reaction buffer; a substrate reaction solution contained 2.5 µM ATP and a biotin-labeled tyrosine kinase substrate diluted to 0.25 µM with the reaction buffer; a detection buffer contained 0.1 ng/µL Eu³⁺ labeled cage antibody (Cisbio, 61T66KLB) and 12.5 nM streptavidin-labeled XI,665 (Cisbio, 610SAXI,B) in the reaction buffer; the compound was dissolved to 10 µM in DMSO, followed by a serial 4-fold dilution with DMSO to a minimum concentration of 0.061 nM. Each concentration was further diluted 40-fold with the reaction buffer.

To a 384-well assay plate (Corning, 3674) were added 4 µL of the compound solutions having a series of concentrations and 2 µL of JAK2 kinase reaction solutions. After being mixed evenly, the mixtures were incubated at room temperature for 15 minutes, and then 4 µL of the substrate reaction solutions were added. The reaction mixtures were incubated at room temperature for 30 minutes. Then the reaction mixtures were added with 10 µL of detection solutions, mixed evenly, and allowed to stand at room temperature for 30 minutes. An Envision plate reader (Perkin Elmer) was then used to measure the progress of the reaction at wavelengths of 620 nm and 665 nm. The signal value (absorbance at 665 nm/absorbance at 620 nm) was positively correlated with the degree of substrate phosphorylation, therefore the kinase activity of JAK2 was detected. In this assay, the group without JAK2 kinase protein was the 100% inhibition group, and the group with JAK2 kinase protein but without the test compound was the 0% inhibition group. The compound inhibition curve was plotted using XLfit software and the IC₅₀ of its inhibition was calculated. The assay results are shown in Table 1.

### Example 15

### TYK2 Activity Inhibition Assay

The effect of the compounds of the present invention on the activity of TYK2 was assessed by using an in vitro kinase detection assay.

The assay method was generally described below:
The enzymatic activity of TYK2 was assessed by detecting the substrate phosphorylation level in a kinase reaction with a homogeneous time-resolved fluorescence (HTRF) kinase detection kit (Cisbio, 62TK0PEC). A reaction buffer contained an enzyme buffer (1×), 5 mM MgCl₂, 1 mM DTT and 0.01%Brij35 from the kit; a human recombinant TYK2 protein (Carna Biosciences, 08-147) was diluted to a kinase reaction solution of 0.25 ng/µL with the reaction buffer; a substrate reaction solution contained 11.25 µM ATP and a biotin-labeled tyrosine kinase substrate diluted to 0.5 µM with the reaction buffer; a detection buffer contained 0.1 ng/µL Eu³⁺ labeled cage antibody (Cisbio, 61T66KLB) and 25 nM streptavidin-labeled XI,665 (Cisbio, 610SAXI,B) in the reaction buffer; the compound was dissolved to 10 µM in DMSO, followed by a serial 4-fold dilution with DMSO to a minimum concentration of 0.061 nM. Each concentration was further diluted 40-fold with the reaction buffer.

To a 384-well assay plate (Corning, 3674) were added 4 µL of the compound solutions having a series of concentrations and 2 µL of kinase reaction solutions. After being mixed evenly, the mixtures were incubated at room temperature for 15 minutes, and then 4 µL of the substrate reaction solutions were added. The reaction mixtures were incubated at room temperature for 40 minutes. Then the reaction mixtures were added with 10 µL of detection solutions, mixed evenly, and allowed to stand at room temperature for 30 minutes. An Envision plate reader (Perkin Elmer) was then used to measure the progress of the reaction at wavelengths of 620 nm and 665 nm. The signal value (absorbance at 665 nm/absorbance at 620 nm) was positively correlated with the degree of substrate phosphorylation, therefore the kinase activity of TYK2 was detected. In this assay, the group without TYK2 kinase protein was the 100% inhibition group, and the group with TYK2 kinase protein but without the test compound was the 0% inhibition group. The compound inhibition curve was plotted using XLfit software and the IC₅₀ of its inhibition was calculated. The assay results are shown in Table 1.

**Table 1**

| Compound No. | JAK2 IC₅₀ (nM) | TYK2 IC₅₀ (nM) |
|---|---|---|
| 1. | 0.3 | 0.2 |
| 2. | 1.9 | 0.2 |
| 3. | 0.4 | 0.1 |
| 4. | 2 | 0.8 |
| 5. | 27 | 4.8 |
| 6. | 5.3 | 0.3 |
| 7. | 62 | 20 |
| 8. | | 220 |
| 9. | 13 | 7 |
| 10. | 4.4 | 0.4 |
| 11. | 1.4 | 04 |
| 12. | 5.5 | 0.9 |
| 13. | 2.2 | 0.3 |
| 14. | 0.5 | 0.3 |
| 15. | 1.3 | 0.2 |
| 16. | 4.9 | 2 |
| 17. | 0.3 | 0.1 |
| 18. | 2.2 | 0.3 |
| 19. | 14 | 23 |
| 20. | 53 | 37 |
| 21. | 12 | 12 |
| 22. | 3.9 | 3.3 |
| 23. | 29 | 50 |
| 24. | 42 | 3.2 |
| 25. | 39 | 5.1 |
| 26. | 2.9 | 0.7 |
| 27. | 1.4 | 0.2 |
| 28. | 0.6 | 0.03 |
| 29. | 0.7 | 0.1 |
| 30. | 0.8 | 0.1 |
| 31. | 0.6 | 0.1 |
| 32. | 0.6 | 0.1 |
| 33. | 0.4 | 0.1 |
| 34. | 0.6 | 0.1 |
| 35. | 0.6 | 0.4 |
| 36. | 0.6 | 0.4 |
| 37. | 0.6 | 0.2 |
| 38. | 0.5 | 0.4 |
| 39. | 4.5 | 0.5 |
| 40. | 2.8 | 0.4 |

The example compounds of the present invention had an inhibition effect on the activities of both JAK2 and TYK2, preferably with an IC₅₀ less than 100 nM, and more preferably with an IC₅₀ less than 10 nM.

### Example 16

### Determination of inhibition of IL-12-induced IFN-γ secretion in NK92 cells

The effect of the compounds of the present invention on IFN-γ secretion in IL-12-induced NK92 cells was evaluated by an enzyme-linked immunosorbent assay (ELISA).

The assay principle was generally described below: IL-12R was mainly expressed in activated T-cells, NK cells (NK92 was an NK cell line), DC cells, and B-cells. When binding to IL-12, it activated the JAK2/TYK2 signal transduction pathway within NK cells and activated T lymphocytes, thereby inducing secretion of IFN-γ.

The assay method was generally described below:
The compound was dissolved to 2.5 mM in DMSO, followed by a serial 4-fold dilution with DMSO to a minimum concentration of 0.31 µM. Each concentration was further diluted 50-fold with an FBS-free MEMα medium (Thermofisher. 12561-056).

NK92 cells (Nanjing Cobioer, CBP60980) were cultured in a complete MEMα medium containing 12.5%FBS (Ausbian, VS500T), 12.5% horse serum (Thermofisher, 16050-122), 0.02 mM folic acid (Sigma, F8758), 0.2 mM inositol (Sigma, 17850), 0.55 mM β-mercaptoethanol (Thermofish, 21985-023), 200 U/mL IL-2 (R&D Systems, 202-1L), and 100 U/mL penicillin-streptomycin mixed liquor (ThermoFisher, 15140122). When covering 80-90% of the culture container surface, the cells were dispersed and plated on a 96-well plate (ThermoFisher, 167425) with 100,000 cells per well (80 µL of the complete MEMα medium without IL-2). The 96-well plate was then incubated overnight in a 37°C, 5%CO₂ incubator.

After overnight incubation, 10 µL of the compound and 10 µL of 50 ng/mL IL-12 (R &D Systems, 219-1L) were added to each well and mixed gently, and the 96-well plate was incubated in the 37°C, 5% CO₂ incubator for additional 24 hours. The plate was centrifuged at 800 rpm for 10 minutes at room temperature and 50 µL of the supernatant from each well was transferred to another 96-well plate (Sigma, CLS3695) coated with anti-IFN-γ antibody. The amount of IFN-γ secretion was detected following the instruction from the Human IFN-γ DuoSet ELISA assay kit (R & D Systems, DY285B). In this assay, the group in which IL-12 and the test compound were replaced with the MEMα medium was the non-stimulated control group (100% inhibition), and the group with IL-12 and 0.2% DMSO was the stimulated control group (0% inhibition). The compound inhibition curve was plotted using XLfit software and the IC₅₀ of its inhibition was calculated. The assay results are shown in Table 2.

**Table 2**

| Compound No. | IC₅₀ (NK92IL12/IFN-γ)(nM) |
|---|---|
| 1 | 57 |
| 2 | 95 |
| 3 | 66 |
| 4 | 175 |
| 5 | 1308 |
| 6 | 198 |
| 10 | 122 |
| 11 | 242 |
| 12 | 110 |
| 13 | 1061 |
| 14 | 113 |
| 15 | 193 |
| 16 | 1443 |
| 17 | 520 |
| 18 | 1406 |
| 26 | 149 |
| 27 | 60 |
| 28 | 38 |
| 29 | 75 |
| 30 | 318 |
| 31 | 56 |
| 32 | 425 |
| 33 | 66 |
| 34 | 65 |
| 35 | 191 |
| 36 | 3477 |
| 37 | 268 |
| 38 | 2854 |
| 39 | 4095 |
| 40 | 406 |

### Example 17

### Pharmacokinetic assay in mice

Test compounds were formulated into a 5 mg/mL dosing sample (suspension or solution) in a 20% HP-β-CD vehicle.

3 fed female C57 mice were each dosed 5 mL/kg by gavage (PO) at a dose of 25 mg/kg. Blood samples were collected 4 hours after dosing, and then animals were killed by CO₂. Colons near the end of the rectum, approximately 4-6 cm in length, were dissected, rinsed with cold saline, blotted dry with absorbent paper, and weighed.

Concentrations of the test compounds in the plasma and intestinal homogenate sample were quantified by LC-MS/MS using an API-4500 mass spectrometer with a plasma limit of quantitation (LOQ) of 1 ng/mL. Pharmacokinetic (PK) parameters were calculated using WinNonlin and the results were summarized in Table 3.

**Table 3**

| Compound | Plasma exposure (ng/mL) | Colon exposure (ng/g) |
|---|---|---|
| 1 | 249 | 5210 |
| 28 | 15 | 2853 |
| 29 | 75 | 3925 |
| 30 | BLOQ | 12200 |
| 34 | 50 | 2705 |
| 36 | 18 | 5093 |

## Claims

1. A compound represented by general formula (I), or a pharmaceutically acceptable salt thereof, a stable isotope derivative thereof, an isomer thereof, or a prodrug thereof: 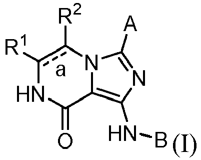 wherein:
bond a is a single bond or a double bond;
R¹ and R² are each independently selected from H, D, CN, C₁₋₆alkyl, and C₃₋₆cycloalkyl, wherein one or more hydrogens of the alkyl are optionally substituted by D or fluorine;
A is C₃₋₁₀cycloalkyl, 4-10 membered heterocyclyl, C₆₋₁₀aryl or 5-10 membered heteroaryl, wherein one or more hydrogens of the cycloalkyl, heterocyclyl, aryl, and heteroaryl are optionally substituted by a substituent selected from D, halogen, cyano, -OR^{a}, -NR^{a}R^{b}, -C(O)R^{a}, -C(O)NR^{a}R^{b}, -S(O)₂R^{a}, - P(O)(CH₃)₂, C₁₋₆alkyl, C₃₋₆cycloalkyl, 4-8 membered heterocyclyl and 5-6 membered heteroaryl;
B is phenyl or 5-6 membered heteroaryl, wherein one or more hydrogens of the phenyl and heteroaryl are optionally substituted by a substituent selected from D, halogen, cyano, -OR^{a}, -NR^{a}R^{b}, -COOR^{a}, - C(O)R^{a}, -NR^{a}C(O)R^{b}, -C(O)NR^{a}R^{b}, -S(O)₂R^{a}, -S(O)₂NR^{a}R^{b}, -S(O)(NR^{a})R^{b}, -P(O)(CH₃)₂ and R¹¹;
R¹¹ is C₁₋₆alkyl, C₃₋₆cycloalkyl, 4-8 membered heterocyclyl or 5-6 membered heteroaryl, wherein one or more hydrogens of the alkyl, cycloalkyl, heterocyclyl and heteroaryl are optionally substituted by a substituent selected from D, halogen, CN, -OH, -NH₂, C₁₋₆alkyl, -OC₁₋₆alkyl, -COOR^{a}, -C(O)R^{a} and - C(O)NR^{a}R^{b};
R^{a} and R^{b} are each independently selected from H, C₁₋₆alkyl, C₃₋₆cycloalkyl, and 4-8 membered heterocyclyl, wherein one or more hydrogens of the alkyl, cycloalkyl, and heterocyclyl are optionally substituted by D, halogen or C₁₋₆alkyl;

2. The compound according to claim 1 or a pharmaceutically acceptable salt thereof, a stable isotope derivative thereof, or an isomer thereof, R¹ and R² are both H.

3. The compound according to any one of claims 1-2 or a pharmaceutically acceptable salt thereof, a stable isotope derivative thereof, or an isomer thereof, which is a compound represented by general formula (II): 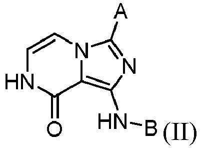 wherein:
A is C₃₋₈cycloalkyl, 4-8 membered heterocyclyl, phenyl or 5-6 membered heteroaryl, wherein one or more hydrogens of the cycloalkyl, heterocyclyl, phenyl and heteroaryl are optionally substituted by a substituent selected from halogen, -C(O)R^{a}, C₁₋₆alkyl and C₃₋₆cycloalkyl;
B is phenyl or 5-6 membered heteroaryl, wherein one or more hydrogens of the phenyl and heteroaryl are optionally substituted by a substituent selected from halogen, -COOR^{a}, -C(O)R^{a}, -C(O)NR^{a}R^{b}, - S(O)₂R^{a}, C₁₋₆alkyl, C₃₋₆cycloalkyl, and 4-8 membered heterocyclyl containing N, S and/or O heteroatom(s), wherein one or more hydrogens of the alkyl, cycloalkyl and heterocyclyl are optionally further substituted by a substituent selected from C₁₋₆alkyl, -C(O)R^{a} and -C(O)NR^{a}R^{b};
R^{a} and R^{b} are each independently selected from H, C₁₋₆alkyl, C₃₋₆cycloalkyl, and 4-6 membered heterocyclyl containing N, S, and/or O heteroatom(s), wherein one or more hydrogens of the alkyl, cycloalkyl, and heterocyclyl are optionally substituted by C₁₋₆alkyl.

4. The compound according to any one of claims 1-3 or a pharmaceutically acceptable salt thereof, a stable isotope derivative thereof, or an isomer thereof, A is phenyl, wherein one or more hydrogens of the phenyl are optionally substituted by halogen.

5. The compound according to claim 1 or a pharmaceutically acceptable salt thereof, a stable isotope derivative thereof, or an isomer thereof, which is a compound represented by general formula (III): 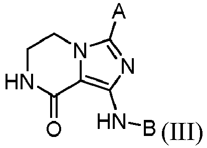 wherein:
A is phenyl, wherein one or more hydrogens of the phenyl are optionally substituted by halogen;
B is phenyl or 5-6 membered heteroaryl, wherein one or more hydrogens of the phenyl and heteroaryl are optionally substituted by a substituent selected from halogen, -COOR^{a}, -C(O)R^{a}, -C(O)NR^{a}R^{b}, - S(O)₂R^{a}, C₁₋₆alkyl, C₃₋₆cycloalkyl, and 4-8 membered heterocyclyl containing N, S and/or O heteroatom(s), wherein one or more hydrogens of the alkyl, cycloalkyl and heterocyclyl are optionally further substituted by a substituent selected from C₁₋₆alkyl, -C(O)R^{a} and -C(O)NR^{a}R^{b};
R^{a} and R^{b} are each independently selected from H, C₁₋₆alkyl, C₃₋₆cycloalkyl, and 4-6 membered heterocyclyl containing N, S, and/or O heteroatom(s), wherein one or more hydrogens of the alkyl, cycloalkyl, and heterocyclyl are optionally substituted by C₁₋₆alkyl.

6. The compound according to claim 1 or a pharmaceutically acceptable salt thereof, a stable isotope derivative thereof, or an isomer thereof, which compound has a structure of: 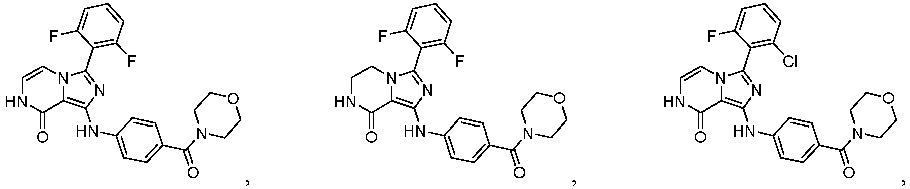 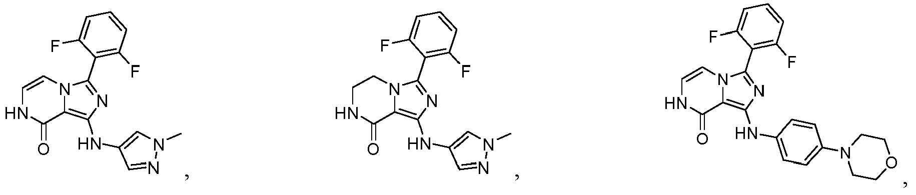 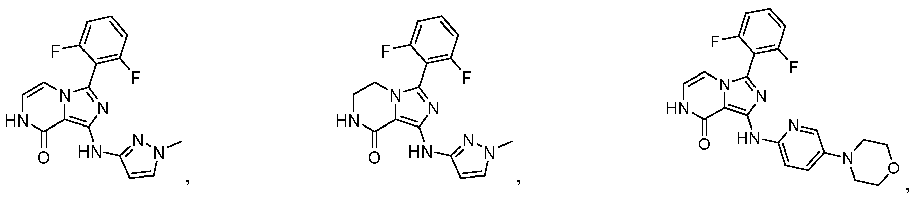 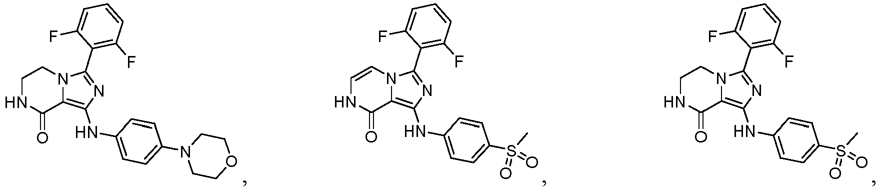 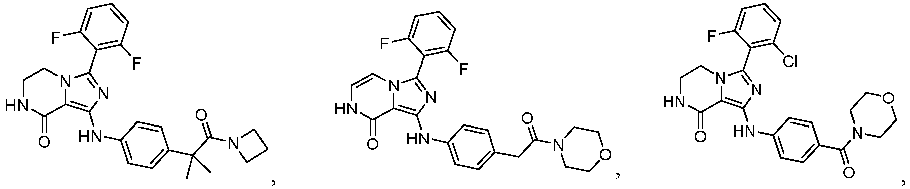 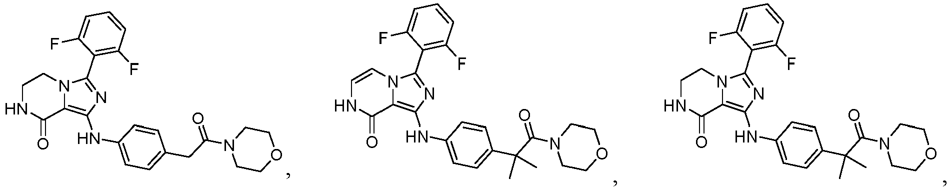 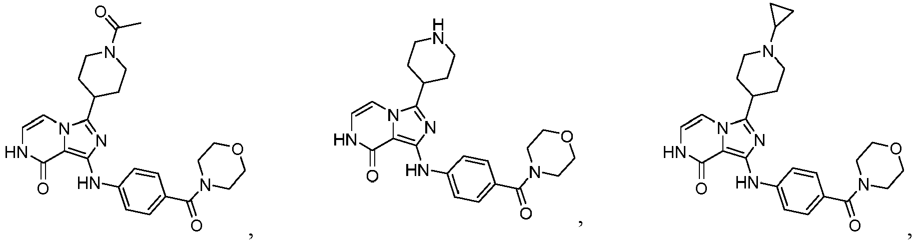 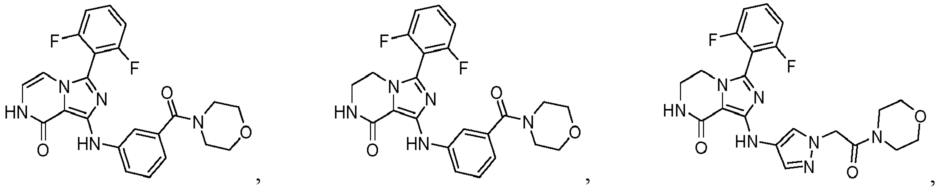 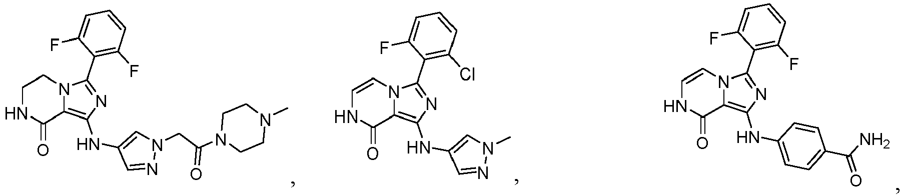 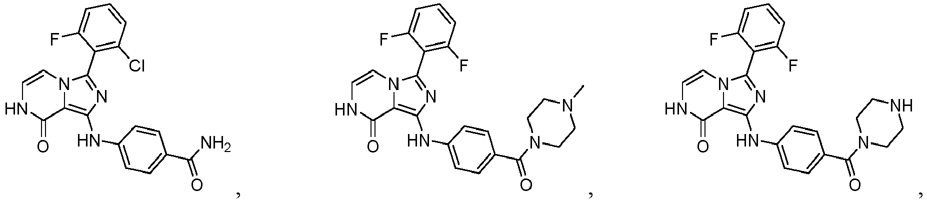 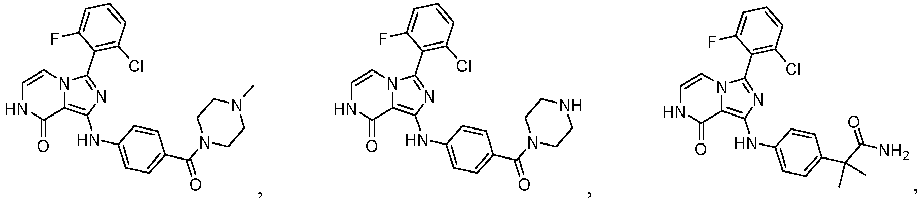 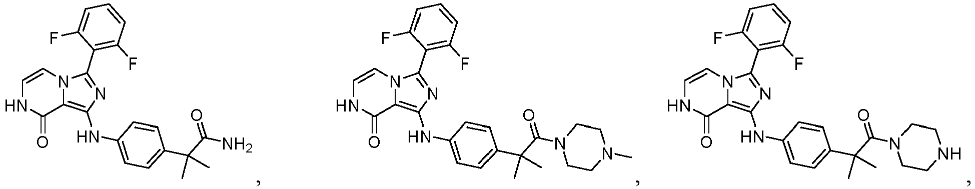 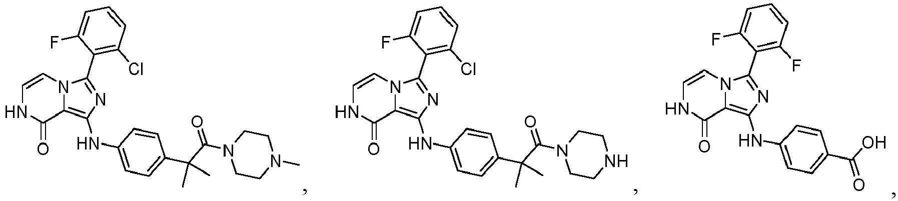 or

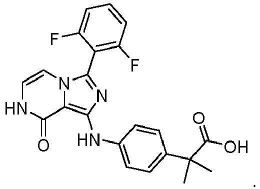

7. The compound of claim 6, which is:

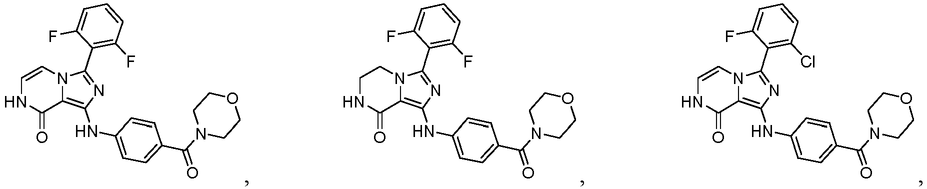

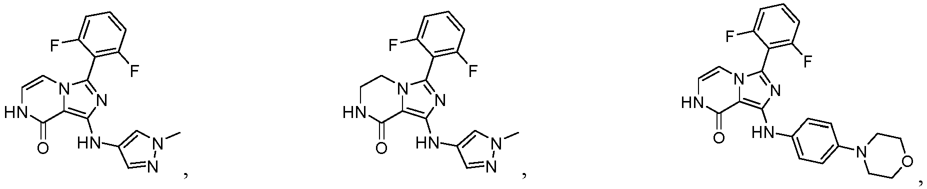

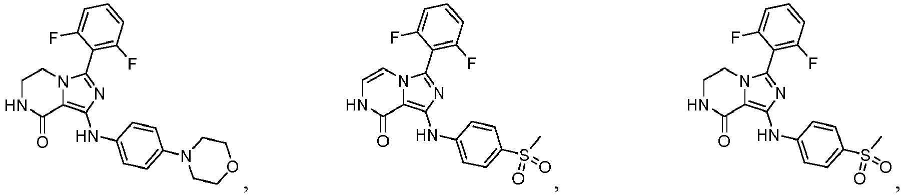

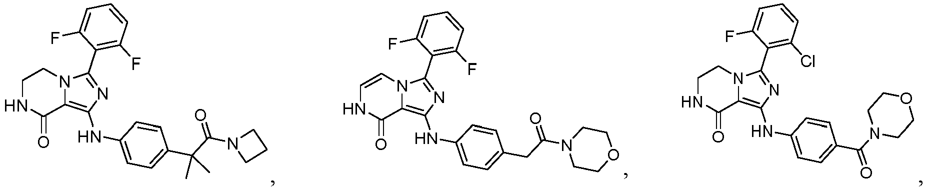

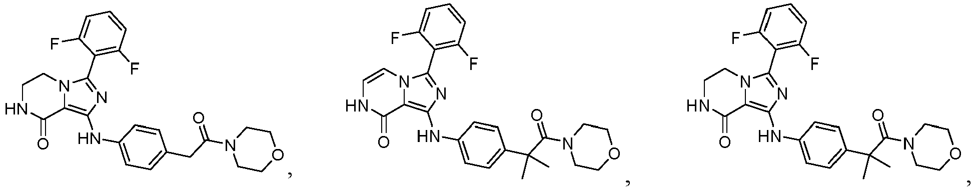

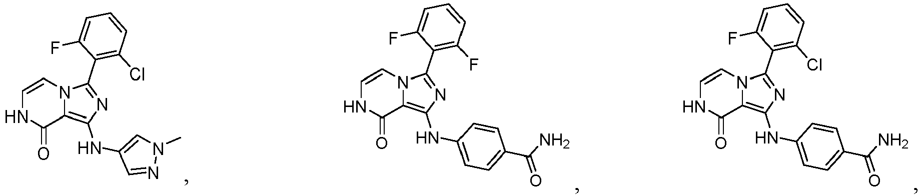

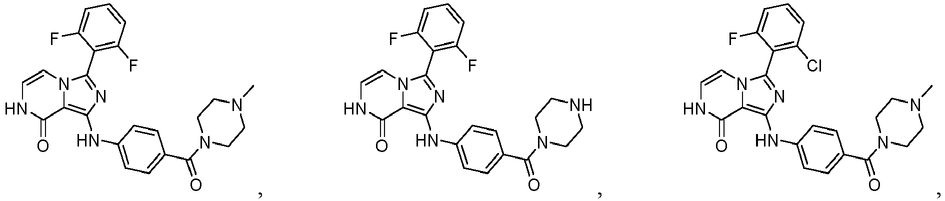

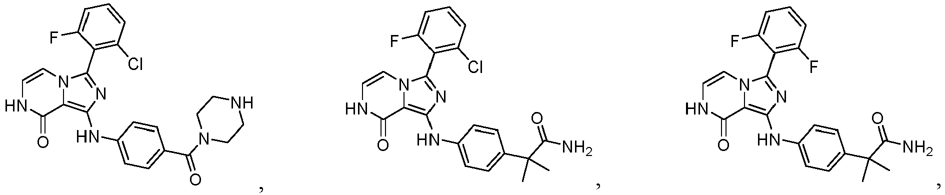

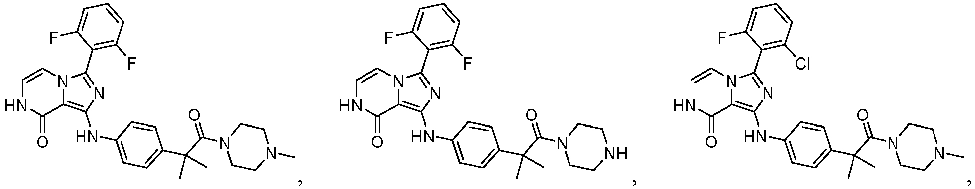

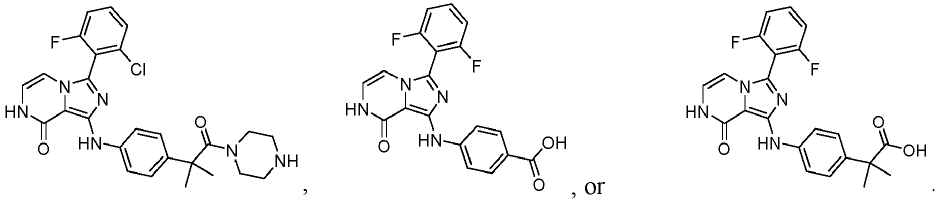

8. A pharmaceutical composition, comprising the compound according to any one of claims 1-7 or a pharmaceutically acceptable salt thereof, a stable isotope derivative thereof, an isomer thereof or a prodrug thereof, and one or more pharmaceutically acceptable carriers or adjuvants.

9. A method for preventing or treating a JAK-mediated related disease, which comprises administering to a patient in need thereof a therapeutically effective amount of the compound according to any one of claims 1-8, or a pharmaceutically acceptable salt thereof, a stable isotope derivative thereof, an isomer thereof or a prodrug thereof, or a pharmaceutical composition comprising said compound, wherein the JAK-mediated disease is an inflammatory disease, an autoimmune disease, a cancer or the like.

10. The method according to claim 9, wherein the disease is inflammatory bowel disease, dermatitis, eczema, rheumatoid arthritis, systemic lupus erythematosus, psoriasis, alopecia areata, or the like.
